# FASCICULE DE BREVET EUROPEEN

(11) **EP 2 397 851 B1**
(45) Date de publication et mention de la délivrance du brevet: **07.01.2015**
(21) Numéro de dépôt: 10166631.1
(22) Date de dépôt: 21.06.2010
(51) Int. Cl.: G01N 33/50, C12N 5/077, C12N 5/0735

(54) **Méthode de sélection des modulateurs de la synthèse de mévalonate en utilisant des cellules dérivées de cellules pluripotentes humaines**
Auswahlmethode der Mevalonat-Synthesemodulatoren unter Einsatz von Derivatzellen pluripotenter menschlicher Zellen
Method for selecting mevalonate synthesis modulators using cells derived from pluripotent human cells

(43) Date de publication de la demande: 21.12.2011
(73) Titulaire: Centre d'Etude des Cellules Souches, 91000 Evry (FR)
(72) Inventeur: Pinset, Christian, 75010, Paris (FR)
(74) Mandataire: Hirsch & Associés

(56) Documents cités:
- WO-A1-2005/111197
- WO-A1-2008/150498
- WO-A1-2009/100760
- WO-A2-2008/094597
- US-A1- 2009 081 784
- KARLSSON C ET AL: "Human embryonic stem cell-derived mesenchymal progenitors-Potential in regenerative medicine", STEM CELL RESEARCH,, vol. 3, no. 1, 1 juillet 2009 (2009-07-01) , pages 39-50, XP026285470, ISSN: 1873-5061, DOI: DOI:10.1016/J.SCR.2009.05.002 [extrait le 2009-05-19]
- BOYD NOLAN L ET AL: "Human Embryonic Stem Cell-Derived Mesoderm-like Epithelium Transitions to Mesenchymal Progenitor Cells", TISSUE ENGINEERING PART A, vol. 15, no. 8, août 2009 (2009-08), pages 1897-1907, XP002609977, ISSN: 1937-3341
- KITAGAWA MICHINORI ET AL: "Differentiation of mesodermal cells from pluripotent stem cells", INTERNATIONAL JOURNAL OF HEMATOLOGY, vol. 91, no. 3, avril 2010 (2010-04), pages 373-383, XP002609978, ISSN: 0925-5710
- DE PEPPO GIUSEPPE MARIA ET AL: "Human Embryonic Mesodermal Progenitors Highly Resemble Human Mesenchymal Stem Cells and Display High Potential for Tissue Engineering Applications", TISSUE ENGINEERING PART A, vol. 16, no. 7, juillet 2010 (2010-07), pages 2161-2182, XP002609979, ISSN: 1937-3341
- EUN J GANG ET AL: "SSEA-4 identifies mesenchymal stem cells from bone marrow", BLOOD, AMERICAN SOCIETY OF HEMATOLOGY, US, vol. 109, no. 4, 15 February 2007 (2007-02-15), pages 1743-1751, XP008152783, ISSN: 0006-4971, DOI: 10.1182/BLOOD-2005-11-010504 [retrieved on 2006-10-24]

## Description

### Domaine de l'invention

La synthèse du mévalonate est impliquée dans de nombreux processus biologiques tels que la synthèse du cholestérol, ainsi que prénylation des protéines. Les différentes étapes de cette voie métabolique ont été identifiées. L'HMG CoA Réductase enzyme limitant pour la synthèse de mévalonate est une cible thérapeutique majeure. Les statines qui sont des inhibiteurs compétitifs de l'HMG CoA Réductase constituent une importante classe de molécules thérapeutiques. La première application clinique de ces molécules est la réduction des lipoprotéines de types LDL dans la circulation générale. Par ailleurs, ces molécules ont des effets pléiotropiques dont certains sont indépendants du contrôle de la synthèse du cholestérol et probablement liés à des modifications secondaires des protéines impliquées dans la signalisation cellulaire. Les statines améliorent la fonction des cellules endothéliales, stabilisent les plaques d'athérome, possèdent des fonctions anti-inflammatoires, jouent un rôle dans la résorption osseuse, diminuent les risques de démence, contrôlent la prolifération cellulaire de certaines cellules normales et tumorales.

D'autre part, les statines présentent une toxicité importante pour certains tissus comme le tissu musculaire ce qui en limite l'utilisation clinique.

Des essais cellulaires permettant le criblage à haut débit de composés interférant avec le métabolisme du mévalonate doivent permettre :
o L'identification de nouveaux inhibiteurs fonctionnels de la synthèse du mévalonate.
o De prévoir des interactions médicamenteuses ayant pour conséquences d'augmenter la toxicité cellulaire, en particulier la toxicité musculaire.
o L'identification de molécules prévenant la toxicité tissulaire en particulier la toxicité musculaire.
o L'identification de molécules prévenant l'atrophie musculaire.
o L'identification de molécules contrôlant sélectivement la prolifération de cellules normales et tumorales, dont les cellules souches tumorales.

La présente invention se rapporte à des procédés de criblage de molécules intervenant dans la synthèse du mévalonate notamment en utilisant des cellules normales différenciées MSC ou de type MSC, dérivées de cellules pluripotentes.

### Résumé de l'invention

L'efficacité du criblage à haut débit, en utilisant des essais cellulaires, est dépendante de la spécificité, de la sensibilité, de la robustesse et de la disponibilité des essais cellulaires utilisées. Cette question est particulièrement importante pour les cellules humaines normales qui sont les modèles les plus adaptés pour le criblage de molécules thérapeutiques pour l'espèce humaine. En effet, la disponibilité des cellules humaines normales est limitée en particulier pour certains types cellulaires comme les cellules cardiaques et neurales. D'autre part, les cellules humaines normales ont une capacité de réplication limitée restreignant leur utilisation pour le criblage à haut débit. Actuellement, la grande majorité des cellules utilisées pour les criblages à haut débit de type HTS sont soit des cellules non humaines, soit des cellules humaines transformées ou tumorales ce qui diminue la pertinence des molécules isolées dans ce type de crible.

La présente invention se rapporte à des procédés de criblage à haut débit de molécules intervenant dans la synthèse du mévalonate en utilisant des cellules normales différenciées MSC ou de type MSC dérivées de cellules pluripotentes. L'invention se rapporte aussi à des procédés de production de cellules normales différenciées dérivées de cellules pluripotentes. L'invention se rapporte également à des procédés de criblage à haut débit utilisant des cellules normales différenciées dérivées de cellules pluripotentes en contrôlant la synthèse de mévalonate par la présence d'inhibiteurs pharmacologiques.

La présente invention à notamment pour objet :
1. Une méthode de production de cellules de type MSC à partir de cellules pluripotentes humaines comprenant une étape de culture des cellules pluripotentes humaines dans un milieu de culture comprenant :
   a. un ou plusieurs facteurs de croissance choisis parmi les FGF, HGF, les PDGF, l'EGF, les hérugulines et les VEGF
   b. un ou plusieurs antioxydants choisis parmi l'acide ascorbique et ses dérivées, la vitamine E et la nacetylcysteine.
2. Une méthode de production de cellules de type MSC à partir de cellules souches pluripotentes induites comprenant une étape de culture des cellules souches induites dans un milieu de culture comprenant :
   a. un ou plusieurs facteurs de croissance choisis parmi les FGF, 1' HGF, les PDGF, l'EGF, les hérugulines et les VEGF
   b. un ou plusieurs antioxydants choisis parmi l'acide ascorbique et ses dérivées, la vitamine E et la nacetylcysteine.
3. Méthode selon l'objet 1 ou 2 caractérisée en ce que le milieu de culture comprend :
   a. du FGF; et
   b. de l'acide ascorbique ou un de ses dérivés.
4. Méthode selon l'objet 3 caractérisée en ce que le milieu de culture comprend :
   a. du FGF-2 ; et
   b. de l'acide ascorbique et/ou de l'acide ascorbique 2 phosphate.
5. Méthode selon l'objet 4 caractérisée en ce que le milieu de culture comprend du FGF2 à une concentration finale de 10ng/ml et de l'acide ascorbique 2 phosphate à une concentration finale de 1mM.
6. Méthode selon les objets précédents caractérisée en ce que les cellules de type MSC produites, expriment un ou plusieurs des marqueurs CD73, CD29, CD44, CD 166 ou CD 105.
7. Méthode selon les objets précédents caractérisée en ce que les cellules de type MSC produites expriment SSEA4
8. Méthode de sélection de composés pharmaceutiques affectant le mévalonate ou le cholestérol, comprenant une étape de mise en contact des cellules obtenues par les procédés des objets 1 à 7 avec les composés pharmaceutiques à tester.
9. Méthode de sélection de composés pharmaceutiques modulant les voies métaboliques du mévalonate ou du cholestérol, comprenant une étape de mise en contact des cellules obtenues par les procédés des objets 1 à 7 avec les composés pharmaceutiques à tester.
10. Méthode de sélection de composés pharmaceutiques accentuant les effets de l'inhibition de la synthèse du mévalonate, comprenant une étape de mise en contact des cellules obtenues par les procédés des objets 1 à 7 avec les composés pharmaceutiques à tester.
11. Méthode de sélection de composés pharmaceutiques compensant les effets de l'inhibition de la synthèse du mévalonate, comprenant une étape de mise en contact des cellules obtenues par les procédés des objets 1 à 7 avec les composés pharmaceutiques à tester.
12. Méthode de sélection de composés pharmaceutiques inhibiteurs fonctionnels de la synthèse du mévalonate et du cholestérol, comprenant une étape de mise en contact des cellules obtenues par les procédés des objets 1 à 7 avec les composés pharmaceutiques à tester.
13. Méthode de sélection de composés pharmaceutiques ayant un effet toxique sur le tissu musculaire, comprenant une étape de mise en contact des cellules obtenues par les procédés des objets 1 à 7 avec les composés pharmaceutiques à tester.
14. Méthode de sélection de composés pharmaceutiques protégeant de l'atrophie musculaire ou de la sarcopénie, comprenant une étape de mise en contact des cellules obtenues par les procédés des objets 1 à 7 avec les composés pharmaceutiques à tester.
15. Méthode de sélection de composés pharmaceutiques antitumoraux, comprenant une étape de mise en contact des cellules obtenues par les procédés des objets 1 à 7 avec les composés pharmaceutiques à tester.
16. Cellules obtenues par les procédés des objets 1 à 7 pour la réparation du tissu osseux.
17. Cellules obtenues par les procédés des objets 1 à 7 pour la réparation du cartilage.
18. Utilisation des cellules obtenues par les procédés des objets 1 à 7 pour l'obtention d'adipocytes blancs ou bruns
19. Utilisation des cellules obtenues par les procédés des objets 1 à 7 pour l'obtention de cellules immunomodulatrices

### Description de l'invention

La présente invention se rapporte à des procédés de criblage à haut débit de molécules intervenant dans la synthèse du mévalonate en utilisant des cellules normales différenciées MSC ou de type MSC dérivées de cellules pluripotentes. Un des objectifs de cette nouvelle méthode est d'augmenter la robustesse, la spécificité et la sensibilité des criblages à haut débit en associant des sources de cellules humaines normales disponibles en quantité illimitée et des modulateurs de la synthèse du mévalonate. La spécificité, la sensibilité, la robustesse et la disponibilité des systèmes cellulaires déterminent la pertinence des criblages à haut débit.

Très fréquemment, les cellules utilisées pour les criblages à haut débit sont des cellules de rongeurs (rat, souris) ou des cellules humaines transformées. À la différence de nombreuses cellules de rongeurs qui présentent des importantes probabilités de transformation spontanée, les cellules humaines normales possèdent des capacités de croissance finie et entrent dans une phase de sénescence après une cinquantaine de divisions cellulaires. La croissance prolongée des cellules humaines s'accompagne d'une instabilité phénotypique. Ces propriétés limitent considérablement l'utilisation des cellules humaines normales pour les criblages à haut débit.

L'existence de cellules souches pluripotentes humaines permet d'avoir accès sans limitations à des cellules humaines normales. Il existe deux types de cellules pluripotentes : les cellules souches embryonnaires (hES) et les cellules souches induites (iPS). Les caractéristiques essentielles des cellules pluripotentes sont la capacité d'auto-renouvellement sans limitation et la possibilité de différenciation en tous les types cellulaires constituant un organisme entier.

La possibilité depuis le travail de J Thompson (Thompson et col 1998) d'utiliser des cellules souches embryonnaires humaines (hES) a ouvert de très nombreuses possibilités. Expérimentalement, les cellules souches embryonnaires humaines ont permis de mettre au point des protocoles de différenciation et d'isoler de nombreux types de cellules (cellules cardiaques, cellules musculaires lisses, cellules neurales, kératinocytes, cellules hématopoïetiques, cellules productrices d'insuline). Cependant, l'isolement de cellules souches embryonnaires est une technologie qui nécessite une importante expertise et qui est dépendante d'un accès à un matériel biologique sensible et limité : les embryons surnuméraires. De plus, les cellules souches embryonnaires soulèvent de nombreuses questions éthiques qui sont résolues différemment selon les états nationaux. Les questions éthiques, réglementaires et de propriétés industrielles limitent actuellement l'utilisation de cellules souches embryonnaires.

La découverte des cellules iPS par l'équipe dirigée par S. Yamanaka (Takahashi K & Yamanaka S 2006) a mis en évidence le caractère réversible de celles-ci et le nombre réduit de gènes dont la co-expression « forcée » peut provoquer la dédifférenciation et de re-programmation de cellules de types très variées en cellules iPS présentant des propriétés très similaires à celles des cellules ES : capacité d'auto renouvellement, de différenciation en tous les types cellulaires formant un organisme et de formation de tératomes une fois injectées chez l'animal tolérant. Il ne semble pas exister de limitations pour l'obtention d'iPS. En effet, des cellules primaires, des cellules immortalisées, des fibroblastes de peau ou de poumons, des hépatocytes, des cellules de pancréas, des lymphocytes et des précurseurs neuraux ont pu être « reprogrammés » en cellules iPS. Des cellules iPS issues de cellules de patients porteurs de nombreuses pathologies comme SMA, ALS, Parkinson, ou Dystrophie de Duchenne ont également été produites (Park et coll. Et 2008). Cette approche rend donc possible l'isolement de cellules iPS à partir de matériaux biologiques facilement accessibles. Enfin, il faut ajouter que ces très nombreux travaux montrent clairement la robustesse qualitative de cette technologie.

La présente demande se rapporte à la production de cellules différenciées MSC ou de type MSC dérivées de cellules pluripotentes sensibles aux inhibiteurs de l'HMG CoA réductase, enzyme clef dans la synthèse du mévalonate. Des travaux antérieurs ont montré que les cellules dérivées du tissu musculaire, cellules musculaires précurseurs (MPC), présentent une sensibilité particulière aux statines. En effet, la présence de statine inhibe spécifiquement la croissance de ce type de cellules. Le procédé décrit de l'invention permet la production de cellules différenciées sensibles aux inhibiteurs de l'HMG COA réductase dérivées de cellules pluripotentes : cellules hES et cellules iPS. Le procédé de production est un procédé en deux étapes, la première étant un procédé de différentiation à partir de cellules pluripotentes et la seconde étant un procédé d'amplification et de conservation des cellules ainsi différenciées.

Les différentes étapes du procédé de production sont décrites ci-dessous. Le principe de cette méthode est d'initier la différenciation par la dissociation mécanique ou enzymatique des amas de cellules non différenciées provenant de cellules pluripotentes et par l'ensemencement sur un support cellulaire composé de collagène ou de ses dérivées comme la gélatine. Une fois les cellules différenciées, les cellules sont amplifiées dans un milieu comprenant des facteurs de croissance de la famille du FGF et des antioxydants comme les dérivés de l'acide ascorbique. Dans ce but d'autres facteurs de croissance peuvent être utilisés comme l'HGF (Hepatocyte growth factor), les différents PDGF (Platelet Derived Growth Factor), les facteurs de la famille de l'EGF (Epidermal Growth Factor), du VGEF, les herugulines. Avec cette méthode, il est possible de produire des cellules différenciées sans limitations de quantité qui présentent un phénotype stable. Ces cellules présentent un phénotype normal qui se caractérise par un caryotype normal et par une croissance finie.

### Définitions des types cellulaires

### Cellules souches pluripotentes.

Les cellules pluripotentes sont caractérisées par deux propriétés : la capacité à s'auto-renouveller et à former tous les types cellulaires constituant un organisme adulte. Ces cellules en se différenciant peuvent donner des cellules des trois feuillets embryonnaires (ectoderme, endoderme, mésoderme). Il existe essentiellement deux types de cellules pluripotentes les cellules souches embryonnaires et les cellules induites à la pluripotence.

### Les cellules souches embryonnaires (cellules ES).

Les cellules souches embryonnaires sont des cellules souches pluripotentes issues d'un embryon au stade de blastocyste. L'isolement de ces cellules a nécessité d'avoir recours à des embryons. Aujourd'hui, il existe dans le monde de très nombreuses lignées de cellules souches embryonnaires qui sont disponibles.

### Les cellules souches pluripotences induites (cellules iPS)

Les cellules souches pluripotentes induites (iPS) sont issues de la reprogrammation de cellules somatiques adultes en cellules pluripotentes sont pour l'instant dans la grande majorité des cas obtenues par transfert de gènes nécessaires à la reprogrammation. Parmi ces gènes, on trouve des gènes exprimés par les cellules ES comme Sox2 et Oct4 et des gènes contrôlant la prolifération comme cMyc, Lin 28 et Klf4. Les cellules iPS possèdent les propriétés essentielles des cellules ES. Dans un futur proche, il va être possible d'obtenir des iPS en utilisant d'autres approches comme le transfert de protéines, le traitement par de petites molécules.

### Cellules mésodermiques progénitrices (MePC)

Les cellules « MSC » sont dérivées de cellules pluripotentes (ES et iPS). Ces cellules appartiennent au feuillet mésodermique sont dépendantes pour leur croissance de l'attachement à un substrat et présentent du capacité de croissance importante mais finie. Les cellules « MSC » expriment une série de marqueurs membranaires comme CD44, CD29, CD73, CD105 et SSEA4 et sont dans certaines conditions expérimentales capables de former du tissu osseux, du tissu cartilagineux et du tissu adipeux.

### Cellules mésodermiques stromales ou souches (Cellules MSC).

Chez l'adulte, les MSC peuvent être isolées de très nombreux tissus. Les premières cellules MSC ont été isolées de la moelle osseuse. Dans un deuxième temps des cellules MSC ont été retrouvées dans quasiment tous les tissus étudiés. Ces cellules appartiennent au feuillet mésodermique et sont dépendantes pour leur croissance de l'attachement à substrat et présentent une capacité de croissance finie. Les cellules MSC expriment une série de marqueurs membranaires comme CD44, CD29, CD73 et sont dans certaines conditions expérimentales capables de former du tissu osseux, du tissu cartilagineux et du tissu adipeux.

### Cellules MSC ou de type MSC

Les cellules MSC ou de type MSC signifient les cellules souches mésodermiques aussi connues sous le nom de cellules multipotentes du stroma, ainsi que des cellules ayant les mêmes caractéristiques biologiques. Les cellules MSC ou de type MSC incluent de manière non limitative les Cellules mésodermiques progénitrices (MePC) et les Cellules mésodermiques stromales ou souches du tissu adulte (MSC).

### Cellules musculaires progénitrices (cellules « MPC »).

Les cellules MPC sont issues et isolées du tissu musculaire. Ces cellules sont responsables du maintien de la fonction musculaire et contrôlent la réparation du tissu musculaire. Les MPC sont capables de s'auto-renouveller de manière limitée et de former du tissu musculaire ex vivo et in vivo.

### Description des protocoles d'isolement et de production de cellules pour le criblage dérivées de cellules pluripotentes humaines (hES et iPS).

Les cellules peuvent être produites à partir de cellules hES ou de cellules iPS non différenciées. Les conditions de production sont les suivantes et sont identiques pour les deux types cellulaires. Les îlots de cellules non différenciées qui sont identifiables par leurs caractéristiques morphologiques forment la base du matériel cellulaire pour la production de cellules.

Les îlots de cellules non différenciées identifiables sur des critères morphologiques ou sur des critères d'expression comme l'expression de Tra1-60 ou comme l'expression de la GFP sous le contrôle de promoteur de gène associé au maintien de la pluripotence comme Oct4/pouf 5 sont dissociés mécaniquement sous contrôle de loupe binoculaire à l'aide de d'aiguille. La dissociation enzymatique ménagée en utilisant des enzymes de type collagénase et trypsine peut être aussi utilisée. Il est aussi possible d'ajouter des agents chélateurs des ions divalents comme l'EDTA et l'EGTA. Dans ce dernier cas, l'utilisation d'inhibiteur de la protéine Rhoa (Rock inhibitor) permet un meilleur redémarrage des cellules ainsi dissociées. Les petits amas de cellules obtenues après dissociation sont comptés automatiquement ou à l'aide d'hématocytomètre et leurs viabilités sont estimées en utilisant des techniques d'exclusion de bleu trypan ou de marquage par l'iodure de propidium. L'étape suivante est constituée par l'ensemencement des agrégats cellulaires sur des supports cellulaires recouverts de collagène ou de ses dérivées comme la gélatine. D'autres constituants de la matrice extra cellulaire comme la fibronectine, la laminine, la vitronectine ou des composés synthétiques comme la poly-ornithine peuvent êtres utilisés pour cette étape. Les cellules sont alors cultivées en présence de milieu de culture de type DMEM supplémenté par de la glutamine, de mélange d'acides aminés non essentiel, de béta mercaptoethanol et de sérum de bovins ou d'autre origine comme l'homme. L'opération peut être effectuée en milieu complètement synthétique sans protéine d'origine animale.

Il a pu être montré que l'association de FGF avec des antioxydants comme des dérivés de l'acide ascorbique augmentait à la fois la robustesse du procédé de production, la cinétique de production et la quantité de cellules produites. En utilisant cette approche combinant le FGF avec l'acide ascorbique 2 phosphate, des cellules différenciées dérivées de cellules pluripotentes ont pu être produites dans toutes les expérimentations avec les différents types de cellules pluripotentes que cela soit des cellules hES et des iPS. 5 jours après l'ensemencement, il est possible de distinguer des cellules individuelles adhérentes au substrat cellulaire qui émergent des amas cellulaires puis entrent en divisions cellulaires. Il s'agit de la première étape de différenciation morphologique. Entre le jour 10 et 20, les cellules sont repiquées en utilisant des solutions enzymatiques de type trypsine combinées avec des agents chélateurs de types EDTA. Il est aussi possible d'utiliser la dissociation mécanique. Les cellules ainsi obtenues sont ensemencées sur des supports cellulaires recouverts de collagène ou de ses dérivées de type gélatine. Comme pour la première étape, il est possible d'utiliser d'autre type de substrats. Cette deuxième étape est l'étape d'amplification cellulaire qui permet d'augmenter le nombre de cellules et d'homogénéiser la population cellulaire ainsi obtenue. Dès le premier repiquage la présence de FGF associé à l'acide ascorbique deux phosphate permet d'augmenter le nombre de cellules par un facteur 2, puis, au cours des passages, le nombre de cellules augmentent considérablement en présence de cette combinaison. Le différentiel peut alors atteindre plusieurs log. Il est aussi possible de produire ce type de cellules dans des milieux synthétiques contenant du FGF et des antioxydants comme l'acide ascorbique et ses dérivés. Il est remarquable que la présence de FGF sans antioxydant ne permet pas d'augmenter le nombre de cellules dérivées de cellules pluripotentes. Les cellules ainsi obtenues sont conservées par les procédés classiques comme la cryopréservation avec comme agents de cryopréservation le DMSO. Pour cette étape, il est aussi possible d'utiliser du glycérol, du tréhalose, de la glycine et de l'arbutin comme agents cryoprotecteurs ainsi que des solutions commerciales. La conservation peut être effectuée par vitrification. La cryopréservation des cellules produites à un excellent rendement et ne modifie pas les paramètres biologiques comme la croissance cellulaire ou l'expression de marqueurs de surface. Dans ces conditions de production, les cellules gardent leur caractère normal en présentant une croissance finie et un caryotype normal.

Les procédés de différenciation et d'amplification décrits des cellules ainsi produites présentent une efficacité semblable pour les cellules dérivées d'hES ou d'iPS. Dans les deux cas, la présence de la combinaison du FGF associé à l'acide ascorbique 2 phosphate dans le milieu de culture augmente de manière spectaculaire l'efficacité des procédés de différenciation et d'amplification cellulaire.

### Caractérisation des cellules différenciées dérivées des cellules pluripotentes humaines (hES et IPS) pour le ciblage.

Les premières étapes de caractérisation sont basées sur des critères fonctionnels tels que la capacité de croissance, l'adhésion aux substrats et sur des critères morphologiques. Morphologiquement les cellules produites ont des caractéristiques similaires aux cellules mésenchymateuses. Leur croissance est dépendante de l'attachement à un substrat. Les analyses moléculaires effectuées montrent que l'expression des gènes associés avec l'état de pluripotence caractéristique des cellules pluripotentes comme Oct4, Nanog sont indétectables dans les cellules produites par les techniques de RT-PCR.

L'analyse des marqueurs de surface par cytométrie de flux des cellules produites montrent un absence d'expression de SSEA3 ,de Tra-1-60, de Tra-1-80 qui sont de déterminants membranaires associés avec l'état de pluripotence et une présence de déterminants membranaires comme CD73, CD44, CD166, CD105 et CD29. L'expression de ces marqueurs est aussi présente sur les cellules multipotentes du stroma (MSC). Les cellules MSC ont été individualisées, il y a une dizaine d'années. Les cellules produites par le procédé de l'invention partagent donc certaines propriétés des MSC :
∘ Adhésion aux substrats
∘ Potentiel de croissance élevé mais finie
∘ Conservation par congélation
∘ Homogénéité d'expression pour les marqueurs CD73, CD29, CD44, CD166.
∘ Potentiel de différenciation ostéogénique

### La présence combinée de FGF2 et d'Acide Ascorbique 2 phosphate dans le milieu de culture augmente la capacité de croissance et l'expression de SSEA4 des cellules produites par le procédé de la demande.

Dans le procédé décrit, la présence de FGF2 et d'acide ascorbique 2 phosphate permettent de produire des cellules différenciées à partir de cellules pluripotentes. Cette augmentation des capacités de croissance est associée avec l'expression spécifique d'un marqueur membranaire SSEA4. Ce marqueur membranaire est aussi exprimé par les cellules pluripotentes et certaines cellules progénitrices (neurale ou mésodermique). Il est remarquable de constater que le FGF2 et l'acide ascorbique 2 phosphate isolément ne permettent pas d'induire l'expression de SSEA4 ni d'augmenter de manière importante les capacité de prolifération. Par contre, la présence conjointe du FGF2 et de l'acide ascorbique 2 phosphate augmente considérablement les capacités de croissance et l'expression de SSEA 4 sans modifier l'expression d'autres marqueurs associée à la totipotence comme Tra-1-60, Tra-1 80, SSEA3 et SSEA1. Le procédé décrit, permet de produire de manière homogène des cellules différenciées qui expriment SSEA4. Dans ces conditions de production décrit par le procédé de l'invention, les cellules différenciées possèdent une importante capacité de prolifération qui est associée à l'expression de SSEA4

### Les cellules produites par le procédé décrit, présentent une sensibilité spécifique aux inhibiteurs de la synthèse du mévalonate comme les inhibiteurs de l'HMG-CoA reductase.

Le mévalonate est un précurseur indispensable à la synthèse du cholestérol métabolite clef de l'activité cellulaire. Les statines, inhibiteurs stériques de l'HMG CoA réductase (enzyme qui contrôle la synthèse du mévalonate), bloquent la production de mévalonate et ainsi la production de cholestérol. L'apport de mévalonate par le milieu extra cellulaire permet de restaurer la production de cholestérol malgré la présence de statine.

Ces propriétés permettent de construire des essais cellulaires notamment pour prévoir et analyser la toxicité des inhibiteurs de la synthèse du mévalonate. Certains types cellulaires comme les cellules MPC sont particulièrement sensibles aux statines. Dans ce type cellulaire, les statines sont toxiques et cette toxicité est abolie par la présence de mévalonate dans le milieu extra cellulaire. Il est possible ainsi de montrer par un test cellulaire simple que les statines sont toxiques par un mécanisme qui implique la synthèse du mévalonate.

Des exemples récents avec les inhibiteurs de COX 2 ou de l'HMG-CoA reductase montrent que la sous-estimation des questions toxicologiques peut avoir des conséquences considérables du point de vue, humain, sanitaire et économique. Définir des systèmes de toxicologie prédictive est donc un objectif critique. Les inhibiteurs de l'HMG-CoA reductase dont les statines présentent une toxicité particulière pour le tissu musculaire. Cette toxicité peut aller de simples courbatures à des rhabdomyolyses qui peuvent entraîner la mort.

Les cellules musculaires précurseurs humaines (MPC) sont de bons indicateurs de la toxicité musculaire. En effet, la croissance cellulaire des MPC est inhibée par la présence de statine dans le milieu de croissance. Cette inhibition est dose dépendante. La présence de mévalonate permet de lever totalement l'inhibition de la croissance. Cette inhibition se fait donc au travers l'inhibition de l'HMG CoA reductase enzyme responsable de la production de mévalonate, précurseur de la synthèse du cholestérol.

Les cellules produites à partir de cellules pluripotentes suivant le procédé de l'invention présentent une sensibilité comparable aux MPC pour les inhibiteurs de l'HMG-CoA réductase. En effet, la présence d'inhibiteurs de l'HMG CoA réductase comme la lovostatine ou la simvastatine dans le milieu de culture réduit le nombre de cellules différenciées dérivées de cellules pluripotentes de manière dose dépendante. Cette inhibition est levée par la présence de mévalonate. L'inhibition de la croissance ainsi observée est donc due à l'inhibition de l'enzyme responsable de la production du mévalonate. Les cellules produites à partir de cellules souches embryonnaires et celles produites à partir de cellules pluripotentes induites présentes le même type de sensibilité aux inhibiteurs de l'HMG CoA réductase et au mévalonate.

La technologie des iPS permet de produire des cellules pluripotentes à partir de tous les individus. Le procédé décrit permet de générer des cellules différenciées dérivées des cellules iPS provenant de patients présentant une sensibilité exacerbée aux inhibiteurs de la synthèse du mévalonate. Ces outils cellulaires seront utiles pour comprendre les mécanismes de la toxicité et isoler des marqueurs pour prévenir et suivre cette toxicité

Les cellules produites par le procédé décrit permettent le criblage à haut débit d'inhibiteurs de la synthèse de mévalonate.

Le criblage à haut débit de composés nécessite de pouvoir cultiver les cellules dans des formats compatibles avec cette technologie comme les plaques multipuits de 96 ou de 384 puits. D'autre part, il est aussi nécessaire d'utiliser des systèmes de lectures de résultats rapides compatibles avec les automates d'analyses. Les cellules produites par le procédé de l'invention sont cultivables dans des plaques multipuits de 96 et de 384 puits. Dans ces conditions, les coefficients de variation des numérations de cellules sont faibles. Dans un premier temps, les nombres de cellules obtenues dans les multipuits ont été déterminés par comptage direct des cellules après marquage en utilisant des systèmes d'analyse d'images. Ces systèmes d'analyse sont précis, mais relativement lents. Il a été donc développé des essais basés sur la quantité d'ATP et sur l'activité mitochondriale. Il a pu être démontré que pour les cellules produites par le procédé de l'invention, il existe une relation linéaire entre la quantité d'ATP mesurée ou l'activité mitochondriale et le nombre de cellules par puits. Autrement dit, la quantité d'ATP ou l'activité mitochondriale mesurée est une mesure du nombre de cellules. Une banque des molécules chimiques de plus de 1200 composés représentant les principaux produits actifs utilisés en clinique humaine a été criblée.

Les cellules décrites par le présent procédé ont permis de cribler cette banque. Ces cellules ont été cultivées dans des multipuits de 96 et 384 puits pendant 3 jours en présence et en absence de mévalonate et en présence des différents composés de la banque. Toutes ces expérimentations ont été réalisées avec des automates permettant la distribution des composées et la culture des cellules en milieu stérile. Les composés ont été sélectionnés sur les critères suivants : décroissance du nombre de cellules en absence de mévalonate et maintien du nombre de cellules en présence de mévalonate. Ces critères permettent de trier les molécules dont la toxicité est liée à l'inhibition de la synthèse de mévalonate. Dans tous les criblages effectués, les inhibiteurs de l'HMG CoA reductase enzyme limitant pour la synthèse du mévalonate utilisé en clinique humaine présents dans la banque ont pu être sélectionnés. Ces expérimentations ont pu montrer que les cellules produites selon le présent procédé permettent de cribler les inhibiteurs de la synthèse du mévalonate de manière robuste en utilisant les technologies de criblage à haut débit. Cette preuve de faisabilité permet d'envisager des criblages avec les systèmes ainsi décrits de banques représentant plusieurs centaines de milliers des composés différents. Il faut aussi noter que les molécules sont triées sur un critère fonctionnel, toxicité cellulaire due à l'inhibition de la synthèse de mévalonate. Ces types d'essai vont pouvoir permettre d'isoler d'autres classes d'inhibiteurs de la synthèse du mévalonate. Avec ce système, il est possible d'isoler de nouveaux inhibiteurs sur leur capacité fonctionnelle indépendamment de leur interaction directe avec l'HMG CoA Reductase.

Les cellules produites par le présent procédé permettent le criblage à haut débit de composés protégeant de l'effet toxique des inhibiteurs de la synthèse du mévalonate. Les effets toxiques des inhibiteurs de la synthèse du mévalonate limitent l'utilisation de ce type de composés en clinique. En effet, de nombreux effets secondaires sur le tissu musculaire sont observés dont la plupart sont limités et réversibles. Néanmoins, dans de nombreux cas, autour de 20%, les patients traités présentent des douleurs musculaires (myalgies, crampes). Ces effets entraînent très fréquemment des arrêts de traitement. Identifier des composés pouvant réduire les effets toxiques des inhibiteurs de la synthèse du mévalonate est un objectif qui peut avoir des conséquences cliniques. Dans cette optique, le crible est réalisé en utilisant les cellules produites par le procédé de l'invention qui sont traitées par des inhibiteurs de la synthèse du mévalonate comme les inhibiteurs de l'HMG CoA reductase. Les conditions du crible sont réalisées comme précédemment et les cellules sont traitées avec un inhibiteur de l'HMG CoA reductase, la simvastatine ou toutes molécules de cette classe médicamenteuse. Les composés sont triés sur la capacité à diminuer les effets toxiques de la simvastaine. En utilisant, cette approche une dizaine composé ont pu être sélectionnés sur une banque de plus de 1200 composés. Il s'agit de candidats potentiels pour réduire les effets toxiques de cette classe de molécules thérapeutiques.

D'autre part, il a été montré que les inhibiteurs de l'HMG CoA reductase induisaient l'expression d'une protéine l'atrogine, protéine impliquée dans les phénomènes d'atrophie musculaire. L'atrophie musculaire est associée à de nombreuses pathologies (maladies neuromusculaires, cancer, HIV, insuffisance rénale, vieillissement ou immobilisation) et est par elle même un facteur de mauvais pronostique. Le crible ainsi décrit permet aussi d'isoler des composés pouvant limiter l'atrophie musculaire. En effet, le traitement par des inhibiteurs de l'HMG COA reductase des cellules produites par le présent procédé permet de reproduire dans la boîte de culture un modèle d'atrophie cellulaire contrôlée. Les composés qui limitent la toxicité, pour les cellules produites par le présent procédé, en présence d'inhibiteurs de l'HMGCoa reductase sont des candidats potentiels pour réduire l'atrophie musculaire et traiter la sarcopénie. La sarcopénie est la fonte des muscles due au vieillissement, à une maladie neurologique, à une maladie virale (par exemple le sida) ou à une maladie tumorale. Il s'agit d'une affection fréquente pour laquelle, il n'existe pas encore de solutions thérapeutiques spécifiques.

Les cellules produites par le présent procédé permettent le criblage à haut débit de composés accentuant l'effet toxique des inhibiteurs de la synthèse du mévalonate.

Pouvoir prévoir, les composés accentuant la toxicité cellulaire en présence d'inhibiteurs de la synthèse du mévalonate doit permettre soit de prévenir des associations de médicaments potentiellement toxiques pour les tissus comme le tissu musculaire, soit de définir des associations de médicaments présentant un effet cytotoxiques utiles pour le traitement tumoral. En clinique humaine, il a été fréquemment observé que la toxicité des inhibiteurs de l'HMG Coa reductase pouvait être exacerbée par certaines associations médicamenteuses. D'autre part, depuis quelques années les statines sont utilisées pour leurs propriétés cytotoxiques pour le traitement de certaines formes de tumeurs (tumeurs digestives, tumeurs du sein ou lymphomes) et pour leur possible utilisation comme agents de prévention (cancer du côlon ou cancer de la prostate). Dans la grande majorité des cas, dans cette dernière indication, les statines sont utilisées en association avec d'autres molécules anti-tumorales.

Dans cette optique, le crible est réalisé en utilisant les cellules produites par le procédé décrit qui sont traitées par des inhibiteurs de la synthèse du mévalonate comme les inhibiteurs de l'HMGCOA reductase. Les conditions du crible sont réalisées comme précédemment et les cellules sont traitées avec un inhibiteur de l'HMGCOAreductase, la simvastatine. Les composés sont triés sur la capacité à accentuer les effets toxiques de la simvastaine. En utilisant, cette approche une quinzaine de composés ont pu être sélectionnés sur une banque de plus de 1200 composés. Il s'agit de candidats potentiels pour augmenter les effets toxiques de cette classe de molécules thérapeutiques. Parmi ces composés, certains sont des agents déjà connus pour leur toxicité cellulaire et d'autres sont des agents cytotoxiques utilisées en clinique humaines comme antitumoraux. D'autre part, des données scientifiques et cliniques indiquent qu'il existe des cellules souches tumorales qui sont pour une part responsable des phénomènes de résistance observés aux traitements antitumoraux classiques. Ces cellules « souches » tumorales sont le résultat d'une transition d'un tissu « épithélial » vers un tissu « mésodermique ». La transition vers le mésoderme de cellules épithéliales tumorales donne à ces cellules un avantage de croissance et une plus grande capacité à former des tumeurs. Les cellules « souches » tumorales possèdent certaines caractéristiques communes aux cellules différenciées « MSC » dérivées des cellules pluripotentes. En effet, les cellules « MSC » sont dérivées de cellules pluripotentes qui sont organisées comme un épithélium pour aller vers un tissu mésodermique. Elles subissent au cours de la différenciation une transition du type épithéliale vers le type mésodermique. De plus, les cellules « MSC » expriment à haut niveau le marqueur CD44 qui est aussi exprimé par les cellules « souches » tumorales. Ces arguments permettent de penser que les cellules « MSC » dérivées de cellules pluripotentes représentent un modèle de cellules « souches tumorales » in vitro. Le criblage décrit permet donc d'isoler des composés ayant une activité spécifique contre les cellules « souches » tumorales. Le criblage triant les molécules qui accentuent spécifiquement la toxicité des statines permet d'établir une stratégie rationnelle pour définir les molécules qu'il faut associer aux statines pour augmenter leur potentiel anti-tumoral et pour cibler les cellules « souches tumorales ».

L'utilisation des cellules produites par le présent procédé en présence ou en absence d'inhibiteur de la synthèse du mévalonate, en présence ou en absence de mévalonate, permet de cribler a haut débit des composés pour :
∘ l'inhibition de la synthèse du mévalonate
∘ l'atténuation des effets secondaires des inhibiteurs de l'HMGCOAreductase
∘ prévenir les interactions médicamenteuses ayant des effets de toxicité musculaire
∘ réduire de l'atrophie musculaire
∘ potentialiser les effets cytotoxiques inhibiteurs de l'HMGCOAreductase et définir les associations médicamenteuses pour leur activité antitumorale et en particulier contre les cellules « souches » tumorales.

Les applications thérapeutiques des composés ainsi triés sont très vastes : hypercholestérolémies, atrophies musculaires ainsi que maladies tumorales.

L'exemple 1 ci-dessous est décrit à titre illustratif seulement.

### Exemple 1 Le FGF2 associé à l'acide ascorbique améliorent la robustesse et l'efficacité des techniques de production de cellules différenciées MSC ou de type MSC dérivées de cellules souches embryonnaires humaines (hES) ayant les caractéristiques des cellules multipotentes du stroma (MSC).

### Introduction

Les cellules multipotentes du stroma ou MSC (Multipotent Stroma Cell) ont été individualisées, il y a une dizaine d'années. L'ISCT (International Society for Cell Therapy) défini ces cellules sur plusieurs types de critères :
o Fonctionnels : croissance en culture sur des substrats comme le plastique de culture
o D'identité : absence d'expression de CD45 et de CD34 et expression de CD73, de CD29 et de CD44
o Potentiel de différenciation dans le lignage ostéogénique, chondrocytaire et adipeux.

Dans un premier temps, les MSC ont été isolées de la moelle osseuse. Par la suite, ces cellules ont été purifiées en partie de nombreux tissus comme le tissu adipeux, le sang périphérique, le foie le poumon, le muscle, le placenta, le liquide amniotique ou le sang de cordon. Les MSC sont impliquées dans la réparation tissulaire dont la réparation osseuse, dans l'immunomodulation, dans le contrôle de la réponse angiogénique et dans le phénomène tumoral.

Ces caractéristiques font des MSC des outils cellulaires indispensables pour les études toxicologiques dans la sphère musculosquelettique et pour la découverte de nouvelles voies thérapeutiques dans différents domaines (orthopédie, maladies vasculaires, maladies inflammatoires, cancer et transfert de gènes).

L'isolement et la production de cellules souches à partir de tissus humains présentent cependant de nombreuses limitations :
o Accessibilité des échantillons de tissus humains
o Conditions d'isolement et production qui doivent être adaptés pour chacun des tissus.
o Capacité de croissance limitée.
o Instabilité phénotypique
o Variabilité interindividuelle

L'existence de cellules souches pluripotentes humaines permet d'avoir accès sans limitations à des cellules humaines normales. Il existe deux types de cellules pluripotentes : les cellules souches embryonnaires (hES) et les cellules souches induites (iPS). Les caractéristiques essentielles des cellules pluripotentes sont la capacité d'auto-renouvellement sans limitations et la possibilité de différenciation en tous les types cellulaires constituant un organisme entier.

La possibilité depuis le travail de J. Thompson (Thompson et col 1998) d'utiliser des cellules souches embryonnaires humaines (hES) a ouvert de très nombreuses possibilités dont certaines pourraient avoir des implications cliniques. Les deux caractéristiques essentielles qui sont la capacité d'auto-renouvellement sans limitations et de différenciation dans tous les types cellulaires constituant un organisme adulte en font de ces cellules un outil extrêmement utile. Expérimentalement, les cellules souches embryonnaires humaines ont permis de mettre au point des protocoles de différenciation et d'isoler de nombreux types de cellules (cellules cardiaques, cellules musculaires lisses, cellules neurales, kératinocytes, cellules hématopoïetiques, cellules productrices d'insuline).

### A) Description des protocoles d'isolement et de production de cellules de type MSC possédant des caractéristiques communes aux MSC dérivées de cellules souches embryonnaires humaines (HES).

Les cellules sont produites à partir de cellules hES non différenciées.

Les conditions de production sont les suivantes. Les îlots de cellules non différenciées qui sont identifiables par leurs caractéristiques morphologiques sont la base du matériel cellulaire pour la production de ces cellules.
1) Repiquage mécanique des îlots reconnus par la morphologie de cellules hES
   a. Changer les boites de cellules hES pour du milieu EB (voir tableau 1) ;
   b. Strier à l'aiguille puis détacher les agrégats. L'aiguille 29G est montée sur une seringue de 1 ml. Choisir les colonies sans signe de différenciation morphologique. Plusieurs dizaines de colonies sont ainsi isolées. Les colonies sont comptées en présence de bleu trypan pour apprécier leur viabilité ;
   c. Centrifugation à 900 rpm 1 minutes pour culotter sans écraser dans des tubes de 15ml ,
   d. Reprendre le culot dans 1 ml de milieu EB. Aspirer refouler pour dissocier partiellement les agrégats de cellules. La taille des agrégats doit être de plusieurs dizaines de cellules ;
   e. Comptage bleu trypan : Détermination nombre d'agrégats et viabilité.
2) Sélection et amplification des cellules MSC :
   f. Un nombre précis de d'agrégats (plusieurs dizaines) est ensemencé dans le milieu EB sur des boîtes de culture gélatinés. Par flacons de 25 cm² de 50 à 200 agrégats sont ensemencés ;
   g. Le premier changement est effectué après 48h puis tous les 2 à 3 jours. Deux conditions de culture sont testées. Dans la première condition, la source de facteurs de croissance est limitée au sérum de veau foetal (Milieu EB). Pour la seconde, il est ajouté au sérum de veau foetal du FGF2 (10ng/ml) et de l'acide ascorbique 2 phosphate 1mM . Ce milieu est nommé EBMOD.
   h. Après 4 à 5 jours, les cellules sortent des agrégats, s'attachent au substrat et commencent à se diviser.
   i. Entre le jour 10 et 15, les cellules sont repiquées en utilisant une solution de trypsine combinée à l'EDTA .
   j. Après lavage avec du milieu de culture par centrifugation et comptage, les cellules sont ensemencées sur des supports gélatinés d'une densité allant de 2000 cellules à 10 000 cellules par cm².
   k. Les premiers repiquages sont effectués entre le jour 5 et 10.

Dans le cadre de ce protocole, nous avons comparé deux milieux de culture le milieu que nous avons nommés EB et un milieu EBMod auquel nous avons ajouté du FGF2 à la concentration de 10µg/ml et de l'Acide ascorbique 2 Phosphate (« AA2P ») à la concentration de 1mM. La composition du milieu EB est indiquée sur le tableau 1.

**Tableau 1 : Composition du milieu EB**

| | Concentration stock | Concentration finale | Volume (500ml tot) |
|---|---|---|---|
| KO-DMEM | | qsp 500ml | 390 ml |
| SVF (Sérum de veau foetal) | 100% | 20% | 100 ml |
| β-mercaptoéthanol | 50 mM | 50 µM | 500 µl |
| Glutamax | 200 mM | 2 mM | 5 ml |
| NEAA | 100X | 1× | 5 ml |
| (MEM aa non essentielle) | | | |
| Pénicilline/Streptomycine | 10000UI/ml | 10UI/ml | 500µl |

Pour cette étude, les cellules souches embryonnaires hES WT4 (KCL-002), XY ont été utilisées. Cette lignée de cellules hES a été isolée par Stephen Minger du Wolfson Centre for Age-Related Diseases, King's College London. Elle provient de UK Stem Cell Bank: UK Stem Cell Bank .National Institute for Biological Standards and Control. Des résultats similaires sont obtenues avec les cellules souches embryonnaires SA01. Ce protocole est aussi applicable à des cellules souches embryonnaires qui présentent des mutations qui représentent des modèles pathologiques.

### B) Production de cellules différenciées de type MSC partageant des caractéristiques des cellules multipotentes du stroma (MSC) dérivées de cellules souches embryonnaires humaines (hES).

Les deux conditions de milieux ont été analysées sur la production et la caractérisation des cellules obtenues en suivant le protocole décrit précédemment. Dans ce cas les cellules différenciées de type MSC ont été dérivées de cellules souches embryonnaires humaines WT4. Les résultats sont indiqués dans les deux tableaux suivants.

**Tableau 2 : Résultats d'une courbe de croissance obtenus en l'absence de FGF2 et d'AA2P**

| MSWT4 | Nombre de divisions cumulées | Nombre de cellules accumulées |
|---|---|---|
| J 0 | | |
| J 10 | ND | 7.7 10⁵ |
| J 17 | ND | 6.6 10⁵ |
| J 24 | 1.15 | 1.7 10⁶ |
| J 30 | 4.02 | 1.2 10⁷ |
| J 38 | 6.18 | 5.6 10⁷ |
| J 43 | 7.86 | 1.8 10⁸ |
| J 76 | 6.54 | 7.2 10⁷ |

**Tableau 3 : Résultats obtenus en présence de FGF2 et AA2P**

| MSC WT 4 FGF2 AA2P | Nombre de divisions cumulées | Nombre de cellules cumulées |
|---|---|---|
| J0 | | |
| J 10 | ND | 1.5 10⁶ |
| J 17 | 2.96 | 1.2 10⁷ |
| J 21 | 4.54 | 3.6 10⁷ |
| J 23 | 6.1 | 1 10⁸ |
| J 30 | 9.6 | 1.2 10⁹ |
| J 38 | 13.3 | 1.6 10¹⁰ |
| J 43 | 16.7 | 1.6 10¹¹ |
| J 48 | 21.3 | 4 10¹² |
| J 52 | 24.8 | 4.5 10¹³ |
| J 59 | 30.6 | 2.5 10¹⁵ |
| J 65 | 36 | 1 10¹⁷ |
| J 71 | 40 | 1.8 10¹⁸ |
| J 78 | 43 | 1.4 10¹⁹ |
| J 87 | 44.5 | 3.8 10¹⁹ |
| J 94 | 45.5 | 7.6 10¹⁹ |
| J 101 | 48.6 | 6.5 10²⁰ |
| J 118 | 50.2 | 1.9 10²¹ |

Dans les deux conditions de milieu, il est possible de dériver des cellules adhérentes qui sont capables de se multiplier dans les deux conditions de culture. En absence de FGF et d'acide ascorbique 2 Phosphate, le nombre de cellules isolées est beaucoup moins important. Ces cellules après quelques divisions cellulaires entre en sénescence et sont incapables d'être amplifiées efficacement dans ces conditions. La quantité de cellules obtenues est plus importante en présence du milieu contenant du FGF et de l'acide ascorbique 2 Phosphate. Dans ces dernières conditions, le nombre de cellules peut être augmenté de plusieurs log. L'introduction de cette modification permet aussi de raccourcir les temps de productions des cellules de type MSC. Dans ces conditions, à partir d'une centaine d'agrégats issus de cellules souches embryonnaires humaines, il est possible de produire 10⁸ cellules en 23 jours de cultures qui possèdent un potentiel réplicatif important (plus d'une trentaine de divisions cellulaires). Il faut noter que ces modifications n'altèrent pas la capacité de ces cellules d'entrer dans une phase de sénescence, indiquant le caractère non transformé des cellules ainsi produites. Il faut noter que dans ces conditions, il est possible de produire des cellules « MSC » avec toutes les cellules souches humaines embryonnaires normales ou présentant des mutations. Ce procédé a été appliqué avec succès pour des cellules souches embryonnaires issues d'embryons porteur de la maladie de Steinert. Ces conditions permettent d'augmenter la robustesse du procédé de production de cellules différenciées de type MSC dérivées de cellules souches embryonnaires humaines.

### C) Caractérisation des cellules différenciées de type MSC dérivées de cellules souches embryonnaires humaines (hES) par cytométrie de flux.

La caractérisation des cellules différenciées produites par le procédé de production à partir de cellules souches embryonnaires humaines (hES) a été effectuée par cytométrie de flux. Cette technologie courante permet des analyses qualitatives et quantitatives des molécules présentes à la surface des cellules en utilisant des anticorps spécifiques. Pour cette étape, il a été utilisé des anticorps dirigés contre des marqueurs de membranes associés à l'état de totipotence et contre des marqueurs des cellules MSC. Les cellules ainsi produites n'expriment pas à leurs surfaces, l'antigène TRA 1-60 qui est associées à l'état de totipotence. Des résultats similaires sont obtenus avec des anticorps dirigés contre d'autres marqueurs de la totipotence comme SSEA3 et TRA 1-80. Il s'agit donc de cellules ayant perdu le caractère pluripotent spécifique des cellules souches embryonnaires. Par contre, ces cellules expriment des marqueurs associés aux cellules MSC comme CD73, CD29, CD44, CD166 et CD 105. Les résultats sont obtenus sont montrés dans le tableau 4.

**Tableau 4 :**

| Analyse par cytométrie de flux des marqueurs membranaires de cellules MSC | | |
|---|---|---|
| Anticorps | Intensité de fluorescence | % de cellules positives |
| CD73 | 400 | 99% |
| CD29 | 672 | 100% |
| CD44 | 624 | 100% |
| CD166 | 145 | 98% |
| CD105 | 283 | 95% |
| SSEA-1 | 567 | 1% |

Les cellules produites par le procédé de production sont homogènes à plus de 95 % pour les marqueurs CD73, CD29, CD44, CD166 et CD105. Ces résultats ne sont pas modifiés par la présence de FGF2 et AA2P. Les cellules ainsi produites possèdent les principales caractéristiques des cellules MSC pour leurs marqueurs de surface.

### D) La présence combinée de FGF2 et d'Acide Ascorbique 2 phosphate dans le milieu de culture augmente la capacité de croissance et l'expression de SSEA4 des cellules produites par le présent procédé.

Le rôle du FGF2 et de l'acide ascorbique 2 phosphate sur les capacités de croissance des cellules isolées suivant le présent procédé ont été analysée sur des cellules dérivées des cellules souches embryonnaires humaines SA01. Les cellules sont cultivées en présence de sérum de veau fétal a 20% (condition de contrôle) et en présence ou en absence de FGF2 et d'acide ascorbique 2 phosphate (AA2P) Les résultats qui représentent le nombre de cellules maximales obtenues avant l'entrée en sénescence sont indiqués sur le tableau 5 suivant.

**Tableau 5 :**

| | Control (FCS20%) | Control (FCS20%) FGF2 10ng/ml | Control (FCS20%)AA2P 1mM | Control (FCS20%)FGF2 10ng/ml+ AA2P 1mM |
|---|---|---|---|---|
| Nombre de cellules maximal avant sénescence | 7. 10⁸ | 4.7 10¹³ | 3.7 10¹⁵ | 1.510²⁰ |

La présence combinée de FGF2 et AA2P permet d'augmenter de près de 12 log la quantité de cellules maximales accumulées. Le FGF2 et l'acide ascorbique isolément ont un effet bien moindre sur l'augmentation de la quantité de cellules, un peu moins de 5 log pour le FGF2 et un peu moins de 6 log pour l'acide ascorbique 2 phosphate. L'effet du FGF2 et de l'acide ascorbique est clairement synergique sur la quantité de cellules maximales de cellules accumulées.

Le SSEA4, marqueur membranaire est exprimée sur les cellules pluriotentes et sur un certain nombre de cellules progénitrices de différents tissus comme la moelle, le tissu adipeux ou le tissu neural.

La présence de SSEA4 sur les cellules cultivées dans ces différentes conditions ont été analysées par cytometrie de flux en utilisant un anticorps spécifique dirigé contre SSEA4. Les résultats sont indiqués sur le tableau 6 suivant :

**Tableau 6 :**

| | Control (FCS20%) | Control (FCS20%) FGF2 10ng/ml | Control (FCS20%)AA2P 1mM | Control (FCS20%)FGF2 10ng/ml+ AA2P 1mM |
|---|---|---|---|---|
| Moyene d'intensité de fluorescence | 7 | 10 | 7 | 26 |

Ni le FGF2, ni l'acide ascorbique 2 phosphate n'augmente significativement l'expression de SSEA 4. Par contre, la combinaison des deux molécules augmente la quantité de SSEA4 d'un facteur 3.7. Ce niveau de fluorescence est comparable à celui observée avec les cellules pluripotentes humaines. La présence combinée de FGF2 et d'acide ascorbique 2 phosphate augmente de manière combinée le nombre maximal de cellules accumulées et le niveau d'expression de SSEA4.

### E) Les cellules de type MSC produites suivant le présent procédé possèdent un caryotype normal.

Le caryotype des cellules « MSC » a été analysé sur des chromosomes en métaphase et s'est révélé sans anomalie détectable en présence ou en absence de FGF2 et AA2P

### F) Les cellules de type MSC produites suivant le présent procédé possèdent un potentiel ostéogénique.

Dans un milieu ostéogénique, les cellules de type MSC produites suivant le présent procédé sont capables d'exprimer des fonctions du tissu osseux . Après 14 jours de culture dans un milieu comprenant de la dexaméthasone , du B glycerophophate les cellules de type MSC produites suivant le présent procédé expriment la phosphatase alcaline et forme des nodules de minéralisation. Les nodules de minéralisation sont révélés par une technique histochimique spécifique la coloration de Von Kossa

### G) Congélation de cellules différenciées de type MSC dérivées de cellules souches embryonnaires humaines (hES).

Les cellules ainsi produites peuvent être conservées par congélation. Le milieu de congélation est le suivant 90% de sérum et 10% de DMSO. La congélation des cellules produites en milieu en présence de FGF2 et AA2P a été pratiquée au passage 7 et à la concentration de 10⁶ par ml.

Les cellules « MSC » produites dans les deux conditions sont très efficacement conservées par congélation. La congélation ne modifie pas les caractéristiques de croissance des cellules « MSC ». Ces techniques de congélation permettent de stocker des grands nombres de cellules sans modifier leurs caractéristiques biologiques.

### H) Conclusions

La présence dans le milieu pour l'amplification des cellules différenciées « MSC » dérivées des cellules hES du FGF2 et de l'acide ascorbique 2 Phosphate permet :
o d'augmenter la robustesse des techniques de production des cellules différenciées à partir des cellules souches embryonnaires
o D'accélérer la production de ces cellules
o D'augmenter le nombre maximal de cellules produites
o D'augmenter le potentiel de prolifération.
o D'obtenir des cellules présentant un caryotype normal
o L'expression spécifique de SSEA4 marqueur de cellules totipotentes et de cellules progénitrices

Les cellules produites dans le milieu en présence de FGF2 et d'acide ascorbique 2 Phosphate possèdent des caractéristiques associées aux cellules MSC :
o Adhésion aux substrats
o Potentiel de croissance élevée mais finie
o Caryotype sans anomalie détectable.
o Conservation par congélation
o Homogénéité d'expression pour les marqueurs CD73, CD29, CD44, CD166, CD 105.
o Potentiel ostéogénique

### Exemple 2 Le FGF2 associé à l'acide ascorbique améliorent la robustesse et l'efficacité des techniques de production de cellules différenciées de type MSC dérivées de cellules pluripotentes humaines (iPS) ayant des caractéristiques des cellules MSC.

### A) Description des protocoles d'isolement et de production de cellules possédant des caractéristiques communes aux MSC à partir cellules humaines pluripotentes induites (IPS).

Les cellules iPS iPS4603C15 et iPS4603PCIA ont été produites à partir de fibroblastes primaires humains en utilisant le protocole de Yamanaka qui introduit les gènes suivant : Oct4, Sox2, cMyc et Klf 4. Le transfert de gènes a été effectué avec des rétrovirus de type Moloney. La transduction virale a été effectuée par une co-infection avec les 4 virus porteurs de chacun des 4 gènes. Le traitement par l'acide valproique a permis d'augmenter l'efficacité de la reprogrammation. Après transduction, les cellules sont cultivées en présence de tissu nourricier constitué de fibroblastes embryonnaires de souris dans un milieu de culture favorable à l'émergence des cellules pluripotentes humaines. Le constituant essentiel du milieu spécifique pour la croissance des cellules humaines pluripotentes est le FGF2. Le milieu est changé toute les 24h. Après deux à trois semaines, les premières colonies ayant les caractéristiques de cellules pluripotentes sont visibles. Ces colonies sont alors isolées et repiquées mécaniquement. Deux populations sont alors constituées : une population monoclonale (iPS iPS4603C15) et une population polyclonale (iPS4603PC1A). Les cellules ainsi isolées possèdent les caractéristiques suivantes :
o Extinction des gènes exogènes transduits utilisé pour la reprogrammation
o Expression des marqueurs de totipotence : SSEA4 et TRA-1-81, TRA 1-60
o Caryotype normal
o Capacité de croissance avec maintien de l'état de pluripotence en milieu en présence de FGF2
o Capacité de différenciation après formation de corps embryonnaires (ectoderme, endoderme, mésoderme)

Ces cellules possèdent les caractéristiques de cellules pluripotentes induites (iPS). Ces cellules ont donc été utilisées pour la production de cellules de type MSC.

Les conditions de production sont les suivantes. Les îlots de cellules non différenciées qui sont identifiables par leurs caractéristiques morphologiques sont la base du matériel cellulaire pour la production de ces cellules.
1) Repiquage mécanique des îlots reconnus par la morphologie de cellules hES
   l. Changer les boites de cellules iPS pour du milieu EB (voir tableau 1).
   m. Strier à l'aiguille puis détacher les agrégats. L'aiguille 29G est montée sur une seringue de 1 ml. Choisir les colonies sans signes de différenciation morphologique. Plusieurs dizaines de colonies sont ainsi isolées. Les colonies sont comptées en présence de bleu trypan pour apprécier leur viabilité.
   n. Centrifugation à 900 rpm 1 minutes pour culotter sans écraser dans des tubes de 15ml
   o. Reprendre le culot dans 1 ml de milieu EB. Aspirer refouler pour dissocier partiellement les agrégats de cellules. La taille des agrégats doit être de plusieurs dizaines de cellules
   p. Comptage bleu trypan : Détermination nombre d'agrégats et viabilité.
2) Sélection et amplification des cellules de type MSC
   q. Un nombre précis d'agrégats (plusieurs dizaines) est ensemencé dans le milieu EB sur des boîtes de culture gélatinée. Par flacons de 25 cm² de 50 à 200 agrégats sont ensemencés.
   r. Le premier changement est effectué après 48h puis tous les 2 à 3 jours. Deux conditions de culture sont testées. Dans la première condition, la source de facteurs de croissance est limitée au sérum de veau foetal (Milieu EB). Pour la seconde, il est ajouté au sérum de veau foetal du FGF2 (1Ong/ml et de l'acide ascorbique 2 phosphate 1mM . Ce milieu est nommé EBMOD
   s. Après 4 à 5 jours, les cellules sortent des agrégats, s'attachent au substrat et commencent à se diviser.
   t. Entre le jour 10 et 15, les cellules sont repiquées en utilisant une solution de trypsine combinée à l'EDTA.
   u. Après lavage avec du milieu de culture par centrifugation et comptage, les cellules sont ensemencées sur des supports gélatinés d'une densité allant de 2000 cellules à 10 000 cellules par cm².
   v. Les premiers repiquages sont effectués entre le jour 5 et 10.

Dans le cadre de ce protocole, nous avons comparé deux milieux de culture le milieu que nous avons nommés EB et un milieu EBMod auquel nous avons ajouté du FGF2 à la concentration de 10µg/ml et de l'Acid ascorbique 2 Phosphate à la concentration de 1mM. La composition du milieu EB est indiquée sur le tableau 1.

**Tableau 7 : Composition du milieu EB**

| | Concentration stock | Concentration finale | Volume (500ml tot) |
|---|---|---|---|
| KO-DMEM | | qsp 500ml | 390 ml |
| SVF | 100% | 20% | 100ml |
| (Sérum de veau foetal) | | | |
| β-mercaptoéthanol | 50 mM | 50 µM | 500 µl |
| Glutamax | 200 mM | 2mM | 5 ml |
| NEAA | 100X | 1× | 5 ml |
| (MEM aa non essentielle) | | | |
| Pénicilline/Streptomycine | 10000UI/ml | 10UI/ml | 500µl |

### B) Production de cellules différenciées partageant des caractéristiques des cellules multipotentes du stroma (MSC) à partir de cellules humaines pluripotentes induites (IPS).

Les deux conditions de milieux ont été analysées sur la production et la caractérisation des cellules obtenues en suivant le protocole identique à celui décrit dans l'exemple 1

Dès le dixième jour, le nombre de cellules observées est plus important dans le milieu en présence de FGF et d'acide ascorbique 2 phosphate. Cette tendance est confirmée au cours de la culture. Les résultats obtenus sont indiqués dans les tableaux suivants.

**Tableau 8 : Courbe de croissance en absence de FGF2 et d'AA2P**

| MSC 4603 CI 5 | Nombre de divisions cumulées | Nombre de Cellules cumulées |
|---|---|---|
| J 0 | ND | ND |
| J 10 | ND | ND |
| J 20 | 2.2 | 1.1 10⁶ |
| J 24 | 4.5 | 5.3 10⁶ |
| J 29 | 7.6 | 4.8 10⁷ |
| J 38 | 12.1 | 1. 10⁹ |
| J 45 | 15.9 | 1.4 10¹⁰ |
| J 52 | 18.2 | 7.4 10¹⁰ |
| J 57 | 20.1 | 2.8 10¹¹ |
| J 66 | 22.2 | 1.1 10¹² |
| J 80 | 23.9 | 3.7 10¹² |
| J 87 | 25.4 | 1 10¹³ |

**Tableau 9 : Courbe de croissance en présence de FGF2 et d'AA2P**

| MSC 4603 Cl 5 FGF2 AA2P | Nombre de divisions cumulées | Nombre de divisions cumulées |
|---|---|---|
| J 0 | ND | ND |
| J 10 | ND | ND |
| J 13 | 0.3 | 9.7 10⁵ |
| J 17 | 2.4 | 4.2 10⁶ |
| J 22 | 7.1 | 1.1 10⁸ |
| J 27 | 11 | 1.6 10⁹ |
| J 31 | 15 | 2.5 10¹⁰ |
| J 38 | 20.6 | 1.2 10¹² |
| J 44 | 25.9 | 4.8 10¹³ |
| J 50 | 31 | 1.7 10¹⁵ |
| J 55 | 35.9 | 5 10¹⁶ |
| J 60 | 38.7 | 3.6 10¹⁷ |
| J 64 | 42.3 | 4.2 10¹⁸ |
| J 71 | 47.3 | 1.3 10²⁰ |
| J 78 | 51.7 | 2.9 10²¹ |
| J 85 | 56.3 | 6.7 10²² |
| J 92 | 59.7 | 7.1 10²³ |

Dans les deux conditions de milieux, il est possible de dériver des cellules adhérentes qui sont capables de se multiplier dans les deux conditions de culture. Il faut cependant remarquer que la quantité de cellules obtenues est plus importante en présence du milieu contenant du FGF et de l'acide ascorbique 2 Phosphate. Dans ces dernières conditions, le nombre de cellules peut être augmenté de plusieurs log. L'introduction de cette modification permet aussi de raccourcir les temps de productions des cellules de type MSC . Dans ces dernières conditions, à partir d'une centaine d'agrégats issus de cellules souches embryonnaires humaines, il est possible de produire 10⁸ cellules en 22 jours de culture qui possèdent un potentiel réplicatif important (plus d'une trentaine de divisions cellulaires).

En absence de FGF2 et de l'acide ascorbique 2 Phosphate, le nombre de cellules isolées est beaucoup moins important. Après trois semaines de culture, le nombre de cellules obtenues est vingt fois moins important. Après 50 jours de culture, le différentiel est de 10⁵. Après une vingtaine de divisions cellulaires, les cellules produites dans ces conditions entrent en sénescence.

Ces résultats obtenus avec un clone de cellules iPS sont confirmés avec une population polyclonales de cellules iPS. Les résultats obtenus avec une population polyclonale sont semblables.

### C) Congélation de cellules différenciées partageant des caractéristiques des cellules de type MSC dérivées de cellules humaines pluripotentes induites (IPS).

Les cellules ainsi produites peuvent être conservées par congélation. Le milieu de congélation est le suivant 90% de sérum et 10% de DMSO. La congélation des cellules produites en milieu EBMod a été pratiquée au passage 7 et à la concentration de 10⁶ par ml. La viabilité est mesurée par un test d'exclusion au bleu trypan.

Les cellules MSC produites dans les deux conditions sont très efficacement conservées par congélation. La congélation ne modifie pas les caractéristiques de croissance des cellules MSC. Ces techniques de congélation permettent de stocker des grands nombres de cellules sans modifier leurs caractéristiques biologiques.

### D) Les cellules « MSC » produites suivant le présent procédé possèdent un caryotype normal.

Le caryotype des cellules « MSC » a été analysé sur des chromosomes en métaphase et s'est révélé sans anomalie détectable en présence en absence de FGF2 et AA2P

### E) Caractérisation des cellules différenciées partageant des caractéristiques des cellules multipotentes du stroma « MSC » à partir de à partir de cellules humaines pluripotentes induites (iPS) par cytométrie de flux.

La caractérisation des cellules différenciées produites par le procédé de production à partir de cellules souches embryonnaires humaines (iES) a été effectuée par cytométrie de flux. Cette technologie courante permet des analyses qualitatives et quantitatives des molécules présentent à la surface des cellules en utilisant des anticorps spécifiques. Pour cette étape, il a été utilisé des anticorps dirigés contre des marqueurs de membranes associées à l'état de totipotence et contre des marqueurs des cellules MSC. Les cellules ainsi produites n'expriment pas à leurs surfaces, l'antigène TRA 1-60 qui est associé à l'état de totipotence. Les autres marqueurs de totipotence comme SSEA3 et TRA 1-80 donnent des résultats semblables. Il s'agit donc de cellules ayant perdu le caractère pluripotent spécifique des cellules souches embryonnaires. Par contre, ces cellules expriment des marqueurs associés aux cellules MSC comme CD73, CD29,CD44 CD166et CD 105
Les résultats obtenus sont montrés dans le tableau 10

**Tableau 10**

| Analyse par cytométrie de flux des marqueurs membranaires de cellules MSC | | |
|---|---|---|
| Anticorps | Intensité de fluorescence | % de cellules positives |
| CD73 | 490 | 93% |
| CD29 | 742 | 93% |
| CD44 | 897 | 99% |
| CD166 | 228 | 97% |
| CD105 | 198 | 98% |
| SSEA-1 | 326 | 1% |

Les cellules produites par le procédé de production sont homogènes à plus de 93 % pour les marqueurs CD73, CD29, CD44, CD166 et CD105. Les cellules ainsi produites possèdent les principales caractéristiques des cellules MSC pour leurs marqueurs de surface.

Des résultats identiques sont obtenus avec les populations de cellules iPS polyclonales. Le protocole ainsi décrit permet avec des résultats similaires d'obtenir des cellules différenciées « MSC » en utilisant des cellules iPS obtenues avec la technique décrite par Thomson. L'efficacité du protocole décrit ne dépend donc pas des techniques de reprogrammation.

### F) La présence combinée de FGF2 et d'Acide Ascorbique 2 phosphate dans le milieu de culture augmente la capacité de croissance et l'expression de SSEA4 des cellules produites par le présent procédé.

SSEA4 marqueur membranaire est exprimée sur les cellules pluriotentes et sur un certain nombre de cellules progénitrices de différents tissus comme la moelle, le tissu adipeux ou le tissu neurale.

La présence de SSEA4 sur les cellules cultivées dans ces différentes conditions ont été analysées par cytometrie de flux en utilisant un anticorps spécifique dirigé contre SSEA4. Les résultats obtenus avec des cellules dérivées de cellules pluripotentes induites à partir de fibroblastes humains (iPS iPS4603C15) sont indiqués sur le tableau suivant :

**Tableau 11**

| 4 | % de cellules positives | Moyenne de Fluorescence |
|---|---|---|
| Control FCS 20% | 35.6 % | 132 |
| Control FCS 20% + FGF2 10ng/ml + AA2P 1mM | 79.6 % | 336 |

La présence de la combinaison de FGF2 et d'acide ascorbique 2 phosphate augmente le nombre de cellules exprimant SSEA4 et l'intensité moyenne de fluorescence.

La présence combinée de FGF2 et d'acide ascorbique 2 phosphate augmente de manière conjointe le nombre maximal de cellules accumulées et le niveau d'expression de SSEA4.

### G) Les cellules de type MSC produites suivant le présent procédé possèdent un potentiel ostéogénique.

Dans un milieu ostéogénique, les cellules de type MSC produites suivant le présent procédé sont capables d'exprimer des fonctions du tissu osseux. Après 14 jours de culture dans un milieu comprenant de la dexaméthasone , du B glycerophophate les cellules de type MSC produites suivant le présent procédé expriment la phosphatase alcaline et forme des nodules de minéralisation. Les nodules de minéralisation sont révélés par une technique histochimique spécifique la coloration de Von Kossa

### H) Conclusions

La présence dans le milieu pour l'amplification des cellules du FGF2 et de l'acide ascorbique 2 Phosphate permet :
∘ d'augmenter la robustesse des techniques de production des cellules différenciées à partir des cellules pluripotentes induites (iPS)
∘ D'accélérer la production de ces cellules
∘ D'augmenter le nombre de cellules produites
∘ D'augmenter le potentiel de prolifération sans modifier leur capacité de sénescence
∘ D'obtenir des cellules présentant un caryotype normal
∘ L'expression spécifique de SSEA4 marqueur de cellules totipotentes et de cellules progénitrices.

Les cellules produites dans le milieu en présence de FGF2 et d'acide ascorbique 2 Phosphate possèdent des caractéristiques associées aux cellules MSC :
∘ Adhésion aux substrats
∘ Potentiel de croissance élevée mais finie.
∘ Conservation par congélation
∘ Homogénéité d'expression pour les marqueurs CD73, CD29, CD44 et CD166.
∘ Potentiel osteogénique

L'exemple 3 ci-dessous est décrit à titre illustratif seulement.

### Exemple 3 : Les cellules différenciées de type MSC dérivées des cellules souches embryonnaires humaines (hES) des outils pour la toxicologie prédictive du tissu musculaire pour les techniques de criblage à haut débit.

### A) Les cellules musculaires précurseurs humaines (MPC) des outils pour la toxicologie prédictive.

Les cellules musculaires précurseurs humaines (MPC) sont de bons indicateurs de la toxicité musculaire. En effet, la croissance cellulaire des MPC est inhibée par la présence de statine dans le milieu de croissance. Cette inhibition est dose dépendante. La présence de mévalonate permet de lever totalement l'inhibition. Cette inhibition se fait donc au travers l'inhibition de l'HMG Coa reductase enzyme responsable de la production de mévalonate précurseur de la synthèse du cholestérol. Avec ce système d'essai cellulaire, la toxicité des différentes statines, ayant été utilisée en clinique humaine, a été testée. Les résultats obtenus sont indiqués dans le brevet : Culture composition, culture method and their uses PCT WO 2004/0055174 A1.

À une concentration de 1µM, l'ensemble des statines présentent une toxicité pour les cellules musculaires. Cette toxicité est aussi dépendante du type de statine. La cérivisatine présente une toxicité plus importante à toutes les concentrations testées. Ces résultats sont en bonne corrélation aux observations cliniques qui ont montré une importante toxicité de la Cerivisatine qui a été retirée du marché pour sa toxicité musculaire importante. Cet essai cellulaire (bioassay) utilisant les MPC a donc une valeur prédictive pour la toxicologie musculaire. Cependant, il existe des limitations intrinsèques à ce type de bioassay qui tiennent aux caractéristiques biologiques des cellules MPC. Les cellules MPC sont des cellules primaires humaines isolées de tissu musculaire qui présentent des variations de lot à lot, des capacités de réplication limitée et une instabilité phénotypique au cours des passages.

### B) Les cellules différenciées de type MSC dérivées des cellules souches embryonnaires humaines comme outils pour la toxicologie prédictive.

L'objectif est donc de remplacer les cellules MPC par des cellules pouvant être produite de manière reproductible en grande quantité et présentant une sensibilité aux inhibiteurs de l'HMGCoA reductase. Les cellules différenciées de type MSC dérivées de cellules humaines pluripotentes (hES et iPS) peuvent être produites sans limitations de nombre (voir exemple 1). Le tableau 2 indique que ces cellules présentent une sensibilité aux statines voisine de celle observée avec les cellules MPC. Les cellules différenciées de type MSC dérivées des cellules hES (SA01) nommées : « MSC » SAMU 43 sont cultivées avec des doses croissantes de Mévoline (Lovostatine) et _de Simvastaine en présence et absence de mévalonate. Après 7 jours de culture, les cellules sont fixées, colorées puis comptées par analyse d'image. Les résultats obtenus sont présentés sur le tableau suivant :

**Tableau 12**

| Courbes dose-réponse des « MSC » SAMU43 : effet du mévalonate | | | | | | |
|---|---|---|---|---|---|---|
| Concentration en M | 0 | 5 10⁻⁶ | 10⁻⁶ | 5 10⁻⁷ | 10⁻⁷ | 5 10⁻⁸ |
| Mevinoline | 9842 | 122 | 3232 | 6934 | 10021 | 10167 |
| Simvastatine | 9553 | 45 | 1750 | 5938 | 10215 | 9467 |

| | | | | | | |
|---|---|---|---|---|---|---|
| Concentration en M | 0 | 5 10⁻⁶ | 10⁻⁶ | 5 10⁻⁷ | 10⁻⁷ | 5 10⁻⁸ |
| Mevinoline +Mevalonate | 8223 | 8148 | 9525 | 9003 | 9423 | 9270 |
| Simvastatine + Mevalonate | 9699 | 9281 | 8991 | 9310 | 8834 | 8408 |

Les cellules « MSC » SAMU43 sont sensibles aux statines de manière dose dépendante. Comme pour les MPC, la présence de mévalonate prévient la toxicité des statines. La toxicité observée est donc due à l'inhibition de synthèse du mévalonate, précurseur du cholestérol. Il est donc possible d'utiliser les cellules différenciées de type MSC dérivées des cellules souches embryonnaires pour évaluer la toxicité cellulaire des inhibiteurs de la synthèse du mévalonate. Ces cellules différenciées de type MSC dérivées de cellules souches embryonnaires constituent donc une source illimitée pour ce type d'essai cellulaire.

### C) Les cellules différenciées de type MSC dérivées de cellules souches embryonnaires des outils pour le criblage .

Le développement de technologie de criblage à haut débit nécessite de pouvoir cultiver les cellules dans des multipuits sans altérer les fonctions biologiques et d'avoir des systèmes de lecture automatisable. Le dosage d'ATP doit pouvoir permettre de déterminer le nombre de cellules de manière automatique.

Dans ce test, l'émission de lumière est proportionnelle à la quantité d'ATP cellulaire. Les expérimentations suivantes ont permis de vérifier la relation entre quantité d'ATP et nombre de cellules. Des nombres croissants de cellules ont été analysés soit par analyse d'image soit par détermination de la quantité d'ATP. Les cellules ont été cultivées dans des multipuits de 96 puits. De 10² cellules à 10⁴ cellules ont été analysées par analyse d'image et par dosage de la quantité d'ATP.

Il existe une bonne linéarité entre le nombre de cellules déterminées par analyse d'image et la quantité d'ATP détectée par luminescence avec un excellent R² de 0.9831. La détermination du nombre de cellules par la quantité d'ATP est donc possible. Cette approche permet une lecture automatisée du nombre de cellules. Des résultats similaires sont obtenus en utilisant des traceurs de l'activité mitochondriale. Les cellules différenciées de type MSC, dérivées des cellules hES, « MSC » WT ont été ensemencées sur des multipuits de 384 préalablement gélatinées en utilisant un automate de distribution de type Bravo, à la concentration de 2000 cellules par puits. La quantité de cellules a été évaluée par le Cell Titer Glow après 72h de culture en présence ou en absence de simvastatine dans un milieu de culture contenant 20% de sérum de veau foetal. Les résultats obtenus sont indiqués dans le tableau 4.

**Tableau 13**

| Toxicité de la simvavstaine pour les MSC dérivées de cellules souches embryonnaires dans des multipuits de 384 puits. | | | | | |
|---|---|---|---|---|---|
| | DMSO | CV DMSO | Statine | CV Statine | DMSO/Statine |
| | 0.2% | 0.2% | 2µM | 2µM | |
| « MSC » | 987206 | 5.4 | 474583 | 15.9 | 2.08 |
| WT | | | | | |

| | | | | | |
|---|---|---|---|---|---|
| CV signifie le coefficient de variation, étant l'Ecart Type divisé par la moyenne. | | | | | |

La simvastatine est toxique pour les cellules différenciées à 2 µM avec un rapport DMSO sur statine supérieur à 2. Les coefficients de variation sont peu élevés, entre 2.9 et 15.9.

La toxicité n'est pas limitée à la simvastatine, mais est aussi observée avec deux autres statines. Dans tous les cas, cette toxicité est abolie par le mévalonate indiquant que cette inhibition se fait au travers de l'inhibition de l'HMGCOA reductase, enzyme responsable de la synthèse du mévalonate. Pour cette expérimentation, les cellules différenciées « MSC » SA01 dérivées des cellules hES (SA01) ont été testées dans des plaques de 384 en utilisant un automate de distribution et la viabilité cellulaire a été mesurée par le Cell Titer Glow après 72h de culture. Les résultats obtenus sont dans le tableau 5 et sont exprimés en pourcentage des résultats obtenus sans traitement.

**Tableau 14**

| Toxicité comparative de statines sur des cellules différenciées « MSC » SA01 dérivées de cellules souches embryonnaires. | | | |
|---|---|---|---|
| | Simvastatine | Lovostatine | Fluvostaine |
| Sans Mévalonate | 48.7% | 53.6 % | 32.8% |
| Avec Mévalonate | 98.3% | 96.4% | 104% |

Les trois statines testées présente une toxicité pour ces cellules et dans tous les cas la présence de mévalonate abolie cette toxicité. Il faut aussi noter que la fluvostatine est plus toxique que la simvastatine et que cette dernière est plus toxique que la lovostatine. Ces résultats sont très semblables a ceux obtenus avec les MPC.

Pour valider expérimentalement les cellules « MSC » dérivées de cellules hES, nous avons cribler une banque de petites molécules, la banque prestwick . Cette banque est constituée de plus de 1200 molécules qui représente la grande majorité des produits utilisés en clinique humaine. Les substances sont utilisées à la concentration de 2 .5µM. Les cellules sont ensemencées dans des multiplaques de 96 puits ou de 384 préalablement gélatinées en présence ou en absence de mévalonate à la concentration de 2mM. 24h plus tard, les composés de la banque sont distribués à l'aide d'un automate de type Velocity. La quantité d'ATP par puits est déterminée à l'aide d'un essai Cell Titer Glow 72 après l'ajout des composées de la banque. Les critères du crible ont été les suivants :
o Une toxicité supérieure à 35% en absence de mévalonate
o Une toxicité inférieure à 20% en présence de mévalonate

Trois essais différents de criblage ont été réalisés. Pour le premier criblage, les cellules « MSC » ont été dérivées des hES humaines SA01. L'essai a été effectué dans 28 multipuits de 96 puits en présence d'un milieu de culture contenant 20% de sérum de veau foetal. La moyenne des Z factor à été de 0.47. Dans ces conditions, il a pu être isoler 11 molécules sur plus de 1200 testées dont les trois statines présentes dans la banque (Lovostatin, Simvastatin, Fluvastatin).

Le deuxième crible a été effectué en utilisant des cellules MSC dérivée d'une autre lignée de cellules souches embryonnaires humaines la lignée VUB01. Dans cet essai, les cellules ont été ensemencées en multi-plaques de 384. Les conditions du crible ont été réalisées comme le précédent. Le Z' Factor moyen a été de 0.55. Le nombre molécules ainsi triées a été de 15 dont les trois statines présentes dans la banque. Un troisième crible a été effectué en plaque 384 avec les mêmes cellules que celle du premier crible. Dans ce cas, le Z'Factor a été de 0.59. Le nombre de molécules ainsi triées a été de trois dont les trois statines contenues dans la banque.

Les résultats obtenus dans les trois cribles indépendants sont représentés et résumés dans le tableau suivant.

**Tableau 15**

| | Cellules | Format | Z' Facteur | Nombre de Hits | Statin |
|---|---|---|---|---|---|
| Crible 1 | « MSC » | 96 | 0.47 | 11 | 3 |
| | SA001 | | | | |
| Crible 2 | « MSC » | 384 | 0.54 | 15 | 3 |
| | VUB001 | | | | |
| Crible 3 | « MSC » | 384 | 0.59 | 3 | 3 |
| | SA001 | | | | |

Les seules molécules que nous avions retrouvées à chaque crible ont été les trois statines contenues dans la banque. On peut donc conclure que les conditions de ce bioassay utilisant les cellules « MSC » dérivées des cellules souches embryonnaires humaines permettent de cribler des molécules inhibant la synthèse du mévalonate de manière sensible et robuste. Les cellules « MSC » dérivées de cellules souches embryonnaires sont des excellents outils pour le criblage à haut débit. Ces propriétés sont observées avec toutes les « MSC » dérivées des cellules hES.

### D) Conclusions

Les cellules différenciées de type MSC dérivées de cellules souches embryonnaires humaines présentent une sensibilité aux statines de même type que les cellules MPC et sont des outils cellulaires robustes, sensibles spécifiques et adaptés pour les techniques de criblage à haut débit de produits ayant une toxicité pour le tissu musculaire et pour les inhibiteurs de la synthèse du mévalonate.

### Exemple 4 : Les cellules différenciées de type MSC dérivées des cellules pluripotentes induites humaines (iPS) comme outils pour la toxicologie prédictive du tissu musculaire pour les techniques de criblage à haut débit.

Les cellules pluripotentes induites iPS qui partagent les caractéristiques essentielles des cellules souches embryonnaires (auto-renouvellement sans limitations et la possibilité de différenciation dans tous les types cellulaires constituant un organisme entier) sont à la fois plus faciles à produire et posent moins de questions réglementaires. Ces propriétés font de ces cellules et de leurs dérivées de bons candidats pour les techniques de criblage à haut débit.

### A) Les cellules différenciées dérivées « MSC » des cellules souches pluripotentes induites humaines des outils pour la toxicologie prédictive dans un criblage à haut débit.

Le développement de technologie de criblage à haut débit nécessite de pouvoir cultiver les cellules dans des multipuits sans altérer les fonctions biologiques et d'avoir des systèmes de lecture automatisable. Le dosage d'ATP doit pouvoir permettre de déterminer le nombre de cellules de manière automatique.

Dans ce test, l'émission de lumière est proportionnelle à la quantité d'ATP cellulaire. Les expérimentations préliminaires ont permis de vérifier la relation entre quantité d'ATP et nombre de cellules. Dans ce type de test, la quantité d'ATP mesurée représente le nombre de cellules.

Les cellules différenciées « MSC » dérivées des cellules souches pluripotentes induites humaines iPS ont été ensemencées à la densité de 2000 cellules par puit de 384 et cultivées pendant 72h en présence et en absence de Simvastatine à la concentration de 2 µM. Les différentes conditions de culture suivantes ont été testées :
o Milieu de culture et Acide ascorbique 2 Phosphate
o Milieu de culture et 1% de sérum de veau foetal
o Milieu de culture et 10% de sérum de veau foetal
o Milieu de culture et 20% de sérum de veau foetal

L'objectif de l'expérimentation a été à la fois de tester la robustesse de l'essai et de définir les conditions optimales pour la réalisation de ce test.

Les résultats chiffrés sont montrés dans le tableau suivant :

**Tableau 16**

| | Simvastatine | CV | DMSO | CV | % Inhibition | Z' Facteur |
|---|---|---|---|---|---|---|
| AA2P | 235 856 | 5,6 | 631 378 | 3 | 63 | 0,76 |
| SVF 1% | 53 212 | 12,2 | 747 366 | 6 | 93 | 0,78 |
| SVF 10% | 294 847 | 14,6 | 1 343 308 | 6,3 | 78 | 0,63 |
| SVF 20% | 471 949 | 14,4 | 1 411 750 | 7 | 67 | 0,76 |

| | | | | | | |
|---|---|---|---|---|---|---|
| CV signifie le coefficient de variation, étant l'Ecart Type divisé par la moyenne. AA2P = Milieu + 1 mMAA2P SVF 1 % =Milieu + 1 % de sérum de veau fetal SVF10% =Milieu + 10% de sérum de veau fetal SVF20% =Milieu + 20% de sérum de veau fetal | | | | | | |

Dans toutes les conditions testées, la simvastatine a un effet toxique important, sur ces cellules, supérieur à 60%. Cette toxicité est très semblable à celle observées avec les cellules musculaires précurseurs (MPC). Il faut aussi noter que dans toutes les conditions expérimentales, le Z'Facteur qui est indicateur statistique de l'effet taille est supérieur à 0,60 ce qui indique que ces essais sont utilisables pour les criblage a haut débit. D'autre part, il est possible de pratiquer ce type d'essai en milieu synthétique sans présence de sérum animal. Dans ce cas, la toxicité observée est de 63 % avec un Z' facteur de 0,76. Les conditions optimales de culture pour ce test sont le milieu en présence de 1% de sérum de veau foetal. Dans ces conditions, le nombre de cellules réduit est de 93% avec un Z'Facteur de 0.78

Pour valider expérimentalement les cellules « MSC » dérivées de cellules iPS, nous avons criblé une banque de petites molécules, la banque prestwick. Cette banque est constituée de plus de 1200 molécules qui représentent la grande majorité des produits utilisés en clinique humaine. Les substances sont utilisées à la concentration de 2 .5 µM. Une représentation schématique du protocole est montrée en annexe 1. Les cellules sont ensemencées dans des multiplaques de 96 puits ou de 384 préalablement gélatinées en présence ou en absence de mévalonate à la concentration de 2mM. 24h plus tard, les composés de la banque sont distribués à l'aide d'un automate de type Velocity. La quantité d'ATP par puits est déterminée à l'aide d'un essai Cell Titer Glow 72 après l'ajout des composées de la banque. Les critères du crible ont été les suivants :
o Une toxicité supérieure à 80% en absence de mévalonate
o Une toxicité inférieure à 50% en présence de mévalonate

L'essai de criblage a été réalisé en utilisant des cellules « MSC » dérivées de cellules IPS. 2000 cellules ont été ensemencées dans 16 plaques de 384 puits en présence et en absence de mévalonate. 24h plus tard, les cellules ont été mis en contact avec les molécules de la banque Prestwic. La mesure a été effectuée après 72 de culture. Le Z' factor de cet essai est supérieur à 0.70.

Avec ces critères, nous identifions uniquement trois molécules qui sont les trois seules statines contenue dans la banque. Ces données montrent qu'il est possible d'utiliser des cellules « MSC » dérivées de cellules iPS pour des criblages à haut débit. Ces dernières cellules possèdent des caractéristiques fonctionnelles très semblables aux cellules « MSC » dérivées des cellules souches embryonnaires.

### B) Conclusions

Les cellules différenciées « MSC » dérivées de cellules pluripotentes induites humaines présentent une sensibilité aux statines de même type que les cellules MPC et sont des outils cellulaires adaptés pour les techniques de criblage à haut débit de produits ayant une toxicité pour le tissu musculaire. Avec ce type de cellules, l'essai cellulaire développé pour le criblage à haut débit est à la fois robuste et sensible. Il faut ajouter qu'il est possible d'effectuer ce test dans des conditions de milieu synthétique et défini.

### Exemple 5. Criblage à haut débit et métabolisme du mévalonate : des applications pour l'atrophie musculaire et pour le cancer.

### A) Introduction

Dans cette série d'essais, l'objectif est d'isoler des molécules pouvant modifier les effets toxiques des inhibiteurs de l'HMG COA reductase. De cette manière, il sera donc possible de trier les molécules sur la capacité à réduire ou à accentuer spécifiquement les effets toxiques des inhibiteurs de l'HMGCOA reductase.

Les molécules réduisant l'effet toxique pourraient être associées aux inhibiteurs de l'HMGCOA reductase pour diminuer les effets délétères de celles-ci sur le tissu musculaire. D'autre part, ces molécules pourront avoir des applications plus étendues dans la protection de l'atrophie musculaire. En effet, il a été montré que dans la grande majorité des atrophies musculaires, la protéine atrogine était augmentée et ce quel que soit l'origine de l'atrophie musculaire. De plus, le traitement des cellules par les inhibiteurs de l'HMGCOA reductase augmente la synthèse de l'atrogine. Il est donc légitime de penser que les cellules traitées par les inhibiteurs de l'HMG COA reductase peuvent constituer un modèle cellulaire in vitro de l'atrophie musculaire. L'atrophie musculaire est une pathologie extrêmement courante qui est associée à de nombreuses pathologies (maladies neuromusculaires, cancer ou VIH). Dans ces dernières pathologies, l'atrophie est un facteur de mauvais pronostique. Prévenir ou réduire l'atrophie musculaire est donc un objectif thérapeutique important.

Les molécules accentuant l'effet toxique sont intéressantes à plus d'un titre. Connaître ces molécules pourrait permettre d'éviter leurs associations thérapeutiques avec les inhibiteurs de l'HMG coa Reductase pour prévenir les effets toxiques potentiels. D'autre part, les inhibiteurs de l'HMGCOA reductase sont utilisés en association avec des molécules anti-cancéreuses pour augmenter leurs potentiels thérapeutiques dans le traitement anti-tumoral. La définition des molécules accentuant les effets des inhibiteurs des l'HMGCOA reductase devra permettre de proposer une base rationnelle pour la définition d'associations thérapeutiques pour les traitements des maladies tumorales.

### B) Principes du criblages de banque de petites molécules avec les cellules différenciées « MSC » dérivées de cellules pluripotentes traitées avec des inhibiteurs de l'HMG COA reductase

Les cellules « MSC » dérivées de cellules pluripotentes sont ensemencées en présence d'inhibiteur de l'HMG Coa Reductase et traitées par la banque de petites molécules. Dans cette expérience, l'inhibiteur de l'HMGCOA reductase utilisé a été la simvastatine à la concentration de 2µM. Après 72 de culture, le nombre de cellules est déterminé pour chaque puit par la technique du Cell Titer Glow. Pour montrer la faisabilité de la technique, la banque de petites molécules Prestwick qui contient plus de 1200 composés dont une grande la grande majorité des principes actifs utilisés en clinique humaine comme pour l'exemple 2 a été choisie. Le processus de travail est schématisé en annexe 2.

### C) Résultats du crible sur des cellules différenciées « MSC » dérivées de cellules humaines pluripotentes traitées par la simvastaine.

Les conditions du criblage ont été :
o 2000 cellules par puits de 384
o J+1 Traitement avec les composés de la banque Prestwick
o J+1 Traitement avec la simvastatine
o J+4 Lecture des résultats avec le Cell Titer Glow.

Le criblage a permis d'identifier deux types de composés. Les premiers ont un rôle protecteur et limite la toxicité des inhibiteurs de l'HMG COA reductase pour les cellules « MSC » . Les seconds exacerbent la toxicité des statines.

Le choix des seuils a été effectué en calculant la moyenne des échantillons et l'écart type. Avec cette méthodologie, une série de composés protecteurs et une série de composés exacerbant la toxicité de la Simvastine ont été déterminées.

Pour les premiers composés, les résultats suivants ont été obtenus qui sont montrés dans le tableau 14.

**Tableau 17 Nombre de composés présentant des moyennes supérieures**

| | M+1ET | M+2ET | M+2.5ET | M+3ET | M+4ET | M+5ET | M+6ET |
|---|---|---|---|---|---|---|---|
| Nombres de Hit | 105 | 11 | 4 | 3 | 2 | 1 | 0 |

| | | | | | | | |
|---|---|---|---|---|---|---|---|
| M = Moyenne des échantillons ET= écart type Nombre de Hit = nombre de molécules présentant une moyenne supérieur à la moyenne+ N écart type | | | | | | | |

11 composés ont une moyenne supérieure à deux écarts types et uniquement 2 composés ont une moyenne supérieure avec 4 écarts types. Les composés ainsi sélectionnés appartiennent à différentes classes médicamenteuses (antihypertenseurs, antihistaminiques, antidépresseurs ou anticholinergique). En utilisant cette approche, il est donc possible d'isoler des composés qui permettent de diminuer les effets toxiques des statines. Une des premières applications des molécules ainsi isolées sera de prévenir les effets délétères des statines et ainsi d'élargir leurs indications cliniques. D'autre part, ces molécules pourront avoir un rôle de protection de l'atrophie musculaire, pathologie très courante pour laquelle les outils thérapeutiques sont encore trop réduits. En effet, les cellules « MSC » traitées par des inhibiteurs de la synthèse du mévalonate représente un modèle dans la boite de culture de l'atrophie musculaire observée dans de nombreuses pathologies primitives (dystrophies musculaires) ou des pathologies secondaires (Sarcopenie, Cancers, HIV, vieillissement). Ce type de criblage permettra de définir des stratégies médicamenteuses pour réduire l'atrophie musculaire ;

Pour la seconde série de composés des composés qui accentuent l'effet des statines, les résultats suivants ont été obtenus qui sont montrés dans le tableau 15.

**Tableau 18 : Nombre de composés présentant des moyennes inférieures**

| | M-1ET | M-2ET | M-3ET | M-4ET | M-5ET | M-6ET |
|---|---|---|---|---|---|---|
| Nombre de Hit | 650 | 203 | 59 | 42 | 32 | 27 |

| | | | | | | |
|---|---|---|---|---|---|---|
| M = Moyenne des échantillons ET= écart type Nombre de Hit = nombre de molécules présentant une moyenne inféreieure à la moyenne+ N écart type | | | | | | |

32 composés présentent une moyenne inférieure à cinq écarts types. Parmi ces 32 composés, il est trouvé de manière attendue les trois statines présentes dans la banque. En effet, la concentration de simvastatine choisie est sous optimale et dans ces conditions il est attendu de mettre en évidence les statines présentes dans la banque. Par une analyse plus fine, il est possible d'isoler des composés dont la toxicité est accentuée est en présence de simvastatine. En effet parmi les 32 composés, 5 composés ne s'étaient pas révélés dans des tests de toxicité en absence de sinvastatine. Il est donc possible d'isoler des composés qui augmentent spécifiquement la toxicité observée en présence de statine. Parmi ces molécules, on trouve des anti-tumoraux et des molécules qui présentent une toxicité musculaire reconnue. Cet essai permet de prédire les molécules qui accentuent la toxicité tissulaire due aux statines. Cette approche permet de prévenir les toxicités dues aux interactions médicamenteuses en présence de statines. Ce test fonctionnel simple à mettre en place pourrait être donc pratiqué de manière systématique pour toutes nouvelles molécules avant le passage en clinique.

La stratégie de criblage est la suivante. Le premier crible permet d'isoler les molécules qui accentuent la toxicité en présence de cellules traitées par les statines. Un contre criblage est alors réalisé sur les molécules ainsi isolées dans le premier crible pour éliminer les molécules qui présentent une toxicité en absence de statines. De cette manière, la spécificité de la toxicité en présence de statine est assurée. Il est significatif qu'avec cette stratégie parmi les 5 molécules triées, 3 sont des antitumoraux.

### C) Conclusions

L'essai cellulaire (bioassay) développé qui utilise comme source cellulaire des cellules différenciées « MSC » dérivées de cellules pluripotentes traitées par un inhibiteur de l'HMG Coa Reductase, la Simvastine, permet de trier des molécules qui protègent de l'effet toxique de simvastine et des molécules qui exacerbent cet effet. Les premières familles de molécules pourront avoir des applications thérapeutiques pour les limitations des effets toxiques des inhibiteurs de l'HMG Coa Reductase et pour prévenir l'atrophie musculaire, pathologie majeure.

Les secondes familles de molécules pourront avoir des applications de toxicologie pour prévoir les interactions médicamenteuses indésirables et applications thérapeutiques dans le traitement tumoral pour déterminer les familles molécules pouvant être combinées avec des inhibiteurs de l'HMG Coa Reductase pour obtenir des effets cytotoxiques sur les cellules tumorales et sur les cellules « souches tumorales ».

## Revendications

1. Méthode de sélection à haut débit de composés pharmaceutiques modulant les voies métaboliques du mévalonate ou du cholestérol, dans laquelle on identifie des composés pharmaceutiques à tester, pouvant décroître le nombre de cellules en absence de mévalonate et maintenir le nombre de cellules en présence de mévalonate comprenant une étape de mise en contact des composés pharmaceutiques à tester avec des cellules obtenues par une méthode de production de cellules de type MSC à partir de cellules souches pluripotentes humaines ou de cellules souches induites, comprenant une étape de culture des cellules souches pluripotentes humaines dans un milieu de culture comprenant : -un ou plusieurs facteurs de croissance choisis parmi les FGF, HGF, les PDGF, l'EGF, les hérugulines et les VEGF -un ou plusieurs antioxydants choisis parmi l'acide ascorbique et ses dérivées, la vitamine E et la nacetylcysteine.

2. Méthode de sélection à haut débit de composés pharmaceutiques compensant ou accentuant les effets de l'inhibition de la synthèse du mévalonate, dans laquelle on identifie des composés pharmaceutiques à tester pouvant réduire ou potentialiser les effets toxiques des inhibiteurs de la synthèse du mévalonate comprenant une étape de mise en contact des composés pharmaceutiques à tester avec des cellules obtenues par une méthode de production de cellules de type MSC à partir de cellules souches pluripotentes humaines ou de cellules souches induites comprenant une étape de culture des cellules souches pluripotentes humaines dans un milieu de culture comprenant : -un ou plusieurs facteurs de croissance choisis parmi les FGF, HGF, les PDGF, l'EGF, les hérugulines et les VEGF -un ou plusieurs antioxydants choisis parmi l'acide ascorbique et ses dérivées, la vitamine E et la nacetylcysteine et lesdites cellules sont traitées avec un inhibiteur de l'HMG CoA reductase.

3. Méthode de sélection à haut débit de composés pharmaceutiques selon la revendication 2, **caractérisée en ce que** l'on utilise des cellules de type MSC obtenues à partir de cellules souches induites, en présence de 1% de sérum de veau foetal.

4. Méthode de sélection à haut débit de composés pharmaceutiques selon la revendication 2, **caractérisée en ce que** l'inhibiteur de l'HMG CoA reductase est la simvastatine ou toutes molécules de cette classe médicamenteuse.

5. Méthode de sélection à haut débit de composés pharmaceutiques selon la revendication 2, comprenant un premier crible permettant d'isoler les composés pharmaceutiques qui accentuent la toxicité en présence de cellules traitées par les statines et un contre criblage réalisé sur les composés pharmaceutiques ainsi isolées dans le premier crible pour éliminer les molécules qui présentent une toxicité en absence de statines.

## Patentansprüche

1. Verfahren zur Selektion mit hohem Durchsatz von pharmazeutischen Verbindungen, um die Verstoffwechselungswege des Mevalonat oder des Cholesterol zu verändern, bei dem die zu testenden pharmazeutischen Verbindungen identifiziert werden, mit denen die Anzahl an Zellen bei fehlendem Mevalonat verringert werden kann und die Anzahl an Zellen bei vorhandenem Mevalonat beibehalten werden kann, einen Schritt umfassend, um die zu testenden pharmazeutischen Verbindungen mit Zellen in Kontakt zu bringen, die durch ein Herstellungsverfahren von Zellen der Art MSC ausgehend von menschlichen pluripotenten Stammzellen oder induzierten Stammzellen erhalten werden, einen Kulturschritt von menschlichen pluripotenten Stammzellen in einem Kulturmilieu umfassend, das Folgendes umfasst:
- einen oder mehrere Wachstumsfaktoren, ausgewählt aus FGF, HFG, den PDGF, dem EGF, den Heregulinen und den VEGF,
- ein oder mehrere Antioxidationsmittel, ausgewählt aus der Ascorbinsäure und ihren Derivaten, dem Vitamin E und dem N-Acetylcystein.

2. Verfahren zur Selektion mit hohem Durchsatz von pharmazeutischen Verbindungen, mit denen die Hemmungseffekte der Synthese des Mevalonat kompensiert oder verstärkt werden, bei dem die zu testenden pharmazeutischen Verbindungen identifiziert werden, mit denen die toxischen Effekte der Synthese-Hemmer des Mevalonat reduziert oder potenziert werden können, einen Schritt umfassend, um die zu testenden pharmazeutischen Verbindungen mit Zellen in Kontakt zu bringen, die durch ein Herstellungsverfahren von Zellen der Art MSC ausgehend von menschlichen pluripotenten Stammzellen oder induzierten Stammzellen erhalten werden, einen Kulturschritt von menschlichen pluripotenten Stammzellen in einem Kulturmilieu umfassend, das Folgendes umfasst:
- einen oder mehrere Wachstumsfaktoren, ausgewählt aus den FGF, HFG, den PDGF, dem EGF, den Heregulinen und den VEGF,
- ein oder mehrere Antioxidationsmittel, ausgewählt aus der Ascorbinsäure und ihren Derivaten, dem Vitamin E und dem N-Acetylcystein, und die Zellen werden mit einem Hemmer der HMG-CoA-Reduktase behandelt.

3. Verfahren zur Selektion mit hohem Durchsatz von pharmazeutischen Verbindungen nach Anspruch 2, **dadurch gekennzeichnet, dass** Zellen der Typs MSC verwendet werden, die ausgehend von Stammzellen erhalten werden, die bei vorhandenem 1 % Kalbföten-Serum induziert werden.

4. Verfahren zur Selektion mit hohem Durchsatz von pharmazeutischen Verbindungen nach Anspruch 2, **dadurch gekennzeichnet, dass** der Hemmer der HMG-CoA-Reduktase das Simvastatin ist oder alle Moleküle dieser Medikamentenklasse sind.

5. Verfahren zur Selektion mit hohem Durchsatz von pharmazeutischen Verbindungen nach Anspruch 2, umfassend eine erste Aussiebung, die ermöglicht, die pharmazeutischen Verbindungen zu isolieren, welche die Toxizität bei vorhandenen Zellen, die mit Statinen behandelt werden, verstärken, und eine Kontroll-Aussiebung, die an den auf diese Weise in der ersten Aussiebung isolierten pharmazeutischen Verbindungen vorgenommen wird, um die Moleküle zu eliminieren, welche eine Toxizität bei fehlenden Statinen aufweisen.

## Claims

1. A method for high-throughput selection of pharmaceutical compounds modulating the metabolic routes of mevalonate or cholesterol, in which pharmaceutical compounds to be tested are identified, and which can decrease the number of cells in the absence of mevalonate and maintain the number of cells in the presence of mevalonate comprising a step of putting the pharmaceutical compounds to be tested in contact with cells obtained by a method for producing cells of the MSC type from human pluripotent stem cells or from induced stem cells, comprising a step of culturing human pluripotent stem cells in a culture medium comprising: one or more growth factors selected from FGFs, HGF, PDGFs, EGF, herugulins and VEGFs, and one or more antioxidants selected from ascorbic acid and its derivatives, vitamin E and N-acetylcysteine.

2. The method for high-throughput selection of pharmaceutical compounds compensating or enhancing for the effects of the inhibition of the synthesis of mevalonate, wherein those compounds to be tested which can reduce or amplify toxic effects of inhibitors of the synthesis of mevalonate are identified, comprising a step of putting the pharmaceutical compounds to be tested in contact with cells obtained by a method for producing cells of the MSC type from human pluripotent stem cells or from induced stem cells comprising a step of cultivating human pluripotent stem cells in a culture medium comprising: one or more growth factors selected from FGFs, HGF, PDGFs, EGF, herugulins and VEGFs, one or more antioxidants selected from ascorbic acid and its derivatives, vitamin E and N-acetylcysteine and said cells are treated with an inhibitor of HMG CoA reductase.

3. The method for high-throughput selection of pharmaceutical compounds according to claim 2, **characterized in that** the cells of the MSC type obtained from induced stem cells are cultivated in the presence of 1% of fetal calf serum.

4. The method for high-throughput selection of pharmaceutical compounds according to claim 2, **characterized in that** the inhibitor of HMG CoA reductase is simvastatin or any molecules of this class of medicaments.

5. The method for high-throughput selection of pharmaceutical compounds according to claim 2, comprising a first screening that allows isolation of the pharmaceutical compounds which enhance toxicity in the presence of cells treated with statins and then a counter-screening is performed on the thereby isolated molecules from the first screening in order to remove the molecules which have toxicity in the absence of statins.
